(19) Europäisches Patentamt / European Patent Office / Office européen des brevets

(11) **EP 4 118 666 B1**

(12) **EUROPEAN PATENT SPECIFICATION**

(45) Date of publication and mention of the grant of the patent:
**28.01.2026 Bulletin 2026/05**

(21) Application number: **21719645.0**

(22) Date of filing: **21.04.2021**

(51) International Patent Classification (IPC):
*H01F 1/08* (2006.01)          *H01F 1/113* (2006.01)
*H01F 1/117* (2006.01)         *H01F 1/28* (2006.01)
*H01F 1/37* (2006.01)          *H01F 1/375* (2006.01)
*H01F 7/02* (2006.01)          *A61B 34/00* (2016.01)
*H01F 13/00* (2006.01)

(52) Cooperative Patent Classification (CPC):
H01F 1/083; A61B 34/72; A61B 34/73; H01F 1/117;
H01F 1/28; H01F 1/375; H01F 7/0215; H01F 13/00

(86) International application number:
**PCT/EP2021/060313**

(87) International publication number:
**WO 2021/219450 (04.11.2021 Gazette 2021/44)**

(54) **METHOD OF FABRICATING PROGRAMMABLE AND/OR REPROGRAMMABLE MAGNETIC SOFT DEVICE, AND METHOD OF ENCODING A PROGRAMMABLE AND/OR REPROGRAMMABLE MAGNETIC SOFT DEVICE**

VERFAHREN ZUR HERSTELLUNG EINER PROGRAMMIERBAREN UND/ODER UMPROGRAMMIERBAREN WEICHMAGNETISCHEN VORRICHTUNG UND VERFAHREN ZUR CODIERUNG EINER PROGRAMMIERBAREN UND/ODER UMPROGRAMMIERBAREN WEICHMAGNETISCHEN VORRICHTUNG

PROCÉDÉ DE FABRICATION D'UN DISPOSITIF MAGNÉTIQUE DOUX PROGRAMMABLE ET/OU REPROGRAMMABLE, ET PROCÉDÉ DE CODAGE D'UN DISPOSITIF MAGNÉTIQUE DOUX PROGRAMMABLE ET/OU REPROGRAMMABLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.04.2020 EP 20171719**

(43) Date of publication of application:
**18.01.2023 Bulletin 2023/03**

(73) Proprietor: **Max-Planck-Gesellschaft zur Förderung der Wissenschaften e.V.**
**80539 München (DE)**

(72) Inventors:
• **ALAPAN, Yunus**
  **70569 Stuttgart (DE)**
• **KARACAKOL, Alp**
  **70569 Stuttgart (DE)**
• **SITTI, Metin**
  **70569 Stuttgart (DE)**

(74) Representative: **Manitz Finsterwald Patent- und Rechtsanwaltspartnerschaft mbB Martin-Greif-Straße 1 80336 München (DE)**

(56) References cited:
**WO-A1-2009/109691**

• **KIM ET AL: "Printing ferromagnetic domains for untethered fast-transforming soft materials", NATURE, vol. 558, no. 7709, 13 June 2018 (2018-06-13), pages 274 - 279, XP036524881, ISSN: 0028-0836, [retrieved on 20180613], DOI: 10.1038/S41586-018-0185-0**

**Description**

The present invention relates to a method of fabricating a programmable and/or reprogrammable magnetic soft device having a Young's modulus of less than 500 MPa in one or more parts of the device. The invention further relates to an untethered programmable and/or reprogrammable, in particular 3D, magnetic soft device having a part with Young's modulus of less than 500 MP, to a method of encoding a programmable and/or reprogrammable magnetic soft device, and to a use of a programmable and/or reprogrammable magnetic soft device.

Shape-changing active matter that can be actuated via external stimuli, such as light, temperature, humidity, pH, and acoustic, electrical and magnetic fields, holds great importance for future applications in minimally invasive medicine, implantable and wearable pieces of equipment, soft robotics, and micromachines. Magnetically responsive soft matter with programmable shape deformation is particularly promising for fast, reversible, and complex morphing of flexible structures for untethered devices.

Shape-programmable magnetic soft matter, composed of magnetic micro/nanoparticles embedded in soft polymers, is promising for the development of untethered (wireless) devices or robots with complex deformation and locomotion capabilities that can operate at small scales, see for instance Kim et al, "Printing ferromagnetic domains for untethered fast-transforming soft materials", Nature, vol. 558.

Magnetic fields generate torque on magnetic soft materials until the magnetization direction of all domains are aligned with the applied field direction. Therefore, creating a spatial distribution of magnetization directions in a magnetic soft material enables programmable shape-deformation under magnetic fields. Current three-dimensional (3D), discrete magnetic programming approaches rely on arranging physical orientation of ferromagnetic particles or alignment of superparamagnetic particles in polymer matrices during curing, which prevents reprogramming once fabricated.

It is an object of the present invention to make available a device by means of which the drawbacks of the prior art are overcome.

This object is satisfied by claim 1, which specifies a method of fabricating a programmable and/or reprogrammable magnetic soft device having a Young's modulus of less than 500 MPa in a part of the device, the method comprising the steps of:

- forming a composite of base material and magnetic elements distributed within said base material;
- shaping the composite to have a desired final shape;
- heating the composite while applying or not applying a magnetic field at the composite; and
- cooling the composite while applying a magnetic field at the composite,

with the step of heating comprising heating the composite to a temperature selected in the range of 25% below the Curie temperature to a temperature 25 % above the Curie temperature measured in °C of said magnetic elements.

In this connection it should be noted that temperatures close to the Curie temperature(Tc) could be used for partial magnetization. i.e. if the sample was already demagnetized it can be magnetized to half strength of the full magnetization of the material of the magnetic elements by only heating to below the Curie temperature.

In this connection a temperature close to or above the Curie temperature of said magnetic elements is selected in the range of 25% below the Curie temperature and to a temperature 25 % above the Curie temperature, in particular to a temperature in the range of 10% below the Curie temperature and to a temperature 10 % above the Curie temperature, most preferably to a temperature in the range of less than 5% below the Curie temperature and to a temperature 5 % above the Curie temperature. Such ranges of temperatures permit a partial magnetization of the respective material, since one can achieve a partial magnetization of 25% to 100% of its max magnetization strength for a demagnetized material already at these temperatures.

The Curie temperature of e.g. Cobalt lies at 1126°C, whereas for $CrO_2$ the Curie temperature lies at 120°C. Depending on whether the material should be partially magnetized one can heat to within 25% of the Curie temperature, i.e. to 250° C below the Curie temperature to 250° C above the Curie temperature for Cobalt. Having regard to $CrO_2$ this partial magnetization state is already achievable at 110°C of the material of the magnetic elements, in particular in the range of 110° to 115°C which is between 5 and 10% below the Curie temperature for $CrO_2$.

Preferably the step of heating is carried out to a temperature below the melting point of the composite respectively of the base material, preferably to a temperature 5° below the melting point of the composite respectively of the base material.

Thus, the present invention makes use of heat-assisted magnetic programming of soft materials by heating the magnetic elements present in the device to above the Curie temperature of the ferromagnetic particles and thereby being able to reorient their magnetic domains with external magnetic fields during cooling. By means of e.g. sequential heat-assisted magnetization over a magnetic soft body, shape-changing instructions in 3D can be encoded discretely and reprogrammed on demand.

Thus, the present invention makes available a versatile strategy for encoding reprogrammable shape-changing instructions into soft materials by encoding the three-dimensional magnetization profile of planar and three-dimensional structures.

The programming approach is based on heating the magnetic soft materials of the composite above the Curie temperature of the ferromagnetic particles and reorienting their magnetic domains by applying an external magnetic field during cooling.

Using heat-assisted magnetic programming, a plethora of flexible structures can be built, including a "dragonfly", a "stickman", magnetic leaves on a non-magnetic tree branches, and microscale "petals", demonstrating discrete, three-dimensional, and reprogrammable magnetization of three-dimensional structures at a high spatiotemporal resolution (currently of approximately 38 $\mu$m).

Using the reprogrammable magnetization capability of the presented approach, the reconfigurable mechanical behavior of an auxetic metamaterial structure, tunable locomotion patterns of a surface walking soft robot, and adaptive grasping behavior of a soft gripper can be shown as will be discussed in the following.

Heat-assisted magnetic programming further enables high-throughput magnetic encoding via contact transfer of distributed magnetization profile from a master, which can go up to 10 samples per minute using a single master. Heat-assisted magnetic programming strategy described here establishes a rich design space and one-shot mass-manufacturing capability for development of multi-scale and reprogrammable soft systems and robots with unprecedented shape-morphing capabilities.

Making available a device that is programmable as well as reprogrammable provides magnetic devices which can be programmed to carry out specific tasks and who can be reprogrammed in the event it is found that fine tuning of parts of the device are required in order to make the device function in an improved or different manner. This was previously not possible, since the devices of the prior art were not reprogrammable.

In this connection it should be noted that a magnetic soft device is a device comprising one or more parts which have a Young's modulus of less than 500 MPa, some parts may have a Young's modulus of less than 100 MPa and some parts may even have a Young's modulus of less than 10 MPa.

In this connection it should also be noted that the average Young's modulus of the magnetic soft device or of parts of the device may be less than 500 MPa, in particular of less than 100 MPa.

This means that the device is generally more flexible than e.g. a device made purely of metal or a rigid plastic such as polyamide (PA), polytetrafluoroethylene (PTFE) and high density polyethylene (HDPE).

In this connection it should be noted that the magnetic elements can be at least one of particles, rods, cubes, wires, disks, spheroids, whiskers, irregular particles, Janus particles, and combinations of the foregoing.

The step of heating may be carried out before, after and/or during the step of shaping the composite. In this way the method can be adapted in correspondence to the materials and the different methods of producing composite materials as required.

The step of shaping and the step of heating may be carried out simultaneously. In this way the production time of the devices can be reduced effectively.

The applied magnetic field during the heating and/or cooling step may be below the coercive magnetic field of the magnetic element at its room temperature state. In this way one prevents magnetization of the undesired regions.

In this connection it should be noted that the applied magnetic field is selected to be within the range of 1 to 99.9% of the coercive magnetic field of the magnetic element at its room temperature state, in particular within the range of 5 to 50% of the coercive magnetic field of the magnetic element at its room temperature state. It should further be noted in this connection that the applied magnetic field could be at least 1 mT.

The step of shaping the composite may comprise at least one of the following steps; molding the composite in one mold of pre-defined shape and size, molding one or more parts of the composite in one or more molds of same shape and size, molding the composite in one or more molds of differing shapes and sizes, photolithographing the composite, photolithographing one or more parts of the composite, stereo lithographing the composite, stereo lithographing one or more parts of the composite, 3D printing the composite, 3D printing one or more parts of the composite, combining parts of the composite, cutting sections of material from the composite, cutting sections of material from parts of the composite and combinations of the foregoing.

In this connection it should be noted that various parts of the device can be manufactured separate from one another and then one can bond the different parts one to another. For example, one could mold the device in eight separate parts and then bond these eight parts to one another. In such cases all eight parts could be magnetized separate from one another. Moreover, some of the parts may be made of non-magnetic materials into which no magnetic elements are introduced, harder or softer materials (in terms of young's modulus), the parts may be made of materials into which different types of magnetic elements are incorporated, the parts may comprise electrically conductive and/or non-conductive materials, piezoelectric materials, magnetocaloric materials, magnetostrictive materials, photovoltaic materials, optoelectronic materials, photomechanical materials, thermoelectric materials, biological materials with and without cultured cells and the parts may comprise combinations of the foregoing.

These parts can be integrated into the device during the molding or printing stage, or separately fabricated parts can be assembled by bonding after their respective manufacture.

In this connection it is feasible to form parts of the composite with a higher density of magnetic elements so that different parts of the composite have different magnitudes of magnetization in addition to different orientations of magnetization.

The melting temperature of the base material may be higher than the maximum temperature applied to the magnetic composite during the heating step. In this way one avoids a melting of the device on programming or reprogramming the device. For example, the melting temperature of the base material is at least 5°C, preferably at least 10°C and most preferably at least 20°C higher than the maximum temperature applied to the magnetic composite during the heating step.

The steps of heating and cooling the composite may be carried out a plurality of times sequentially for different regions of the composite. In this way one device can be scanned over its various regions by the same heating device and magnetic field generating device in order to impart a specific magnetization profile to each region. Preferably each region is only scanned once during the sequential scanning of the plurality of regions.

In this connection it should be noted that if a magnetic master is used then during the method of fabricating a programmable and/or reprogrammable magnetic soft device the steps of heating the composite and cooling of the composite can be carried out only once and these steps are carried out in so to say one-shot. Magnetic masters can be configured to generate arbitrary magnetization fields in neighboring sections and placed nearby the sample to be magnetized. Then heating all sections of the sample will allow magnetization. The magnetic master should possess a Curie temperature above the temperature reached in the step of heating in this process.

In this manner programmable and/or reprogrammable magnetic soft devices having a size selected in the range of 1 $\mu$m to 1 m, in particular of 20 $\mu$m to 30 cm can be produced. Such devices can be used as miniature cargo delivery devices such as drug delivery devices, or for the transport of parcels using e.g. drones or the like.

The step of magnetization may be carried out for each step of cooling for each region of the composite so that each region is provided with its own magnetization direction. In this way one device can be scanned over its various regions by the same heating device and magnetic field generating device in order to impart a specific magnetization profile to each region.

The step of heating the composite may be carried out with a tunable light source, such as a collimated laser. Tunable light sources provide pre-defined heating capabilities which can be configured to reliably and repeatedly work with the magnetic elements present in the composite forming the device. The step of heating the composite may also be carried out with ultrasound, radio frequency electric/electromagnetic radiation, and alternating magnetic fields.

The step of heating the composite may be carried out with one of a convection oven, a hot-plate and a heat-gun. Such apparatus can be reliably used to regionally or completely heat the device during programming and reprogramming of the device.

The steps of heating and cooling the composite may be carried out a single time globally for different regions of the composite, in particular wherein the step of magnetization may be carried out for a single time during cooling for each region of the composite by using a magnetic master configured to generate desired magnetization profile so that each region is provided with its own magnetization direction.

In this connection it should be noted that the step of local and sequential heating, apart from use of a laser, can also be done using a contact-based apparatus, such as a soldering iron tip or other heating devices.

The step of applying the magnetic field may be carried out with a magnetic field having a magnitude selected in the range of 1 mT to 10 T. Such magnetic field strengths enable the programming and reprogramming of the respective regions in an efficient manner.

In this connection it should be noted that the maximum applicable magnetic field depends on the coercive field of the chosen magnetic material.

A further aspect which is not part of the present invention relates to an untethered programmable and/or reprogrammable 3D magnetic soft device having one or more parts with Young's modulus of less than 500 MPa, the programmable and/or reprogrammable 3D magnetic soft device comprising a body formed of a composite material, the composite material comprising a base material and magnetic elements distributed within said base material, wherein the body has an arbitrary magnetization profile, with different regions of the body having different magnetization profiles, wherein the information encoded into the programmable and/or reprogrammable 3D magnetic soft device comprises shape changing instructions for changing a shape of at least some of the regions of the body relative to one another on application of an external field.

The advantages achievable with such a device are the provision of programmable as well as reprogrammable magnetic devices which can be programmed to carry out specific tasks and who can be reprogrammed in the event it is found that fine tuning of parts of the device are required in order to make the device function in an improved or different manner. This was previously not possible, since the device were not reprogrammable.

The base material may be selected from the group of members consisting of elastomers, thermoplastic elastomers, rubbers, duroplastics, thermoplastics, e.g., polydimethylsiloxane, aliphatic aromatic copolyester or modified polyester, or modified copolyester, polyurethane elastomer, silicone rubber, natural rubber, latex, styrene ethylene butylene styrene, butyl rubber, fluorosilicone rubber, polyester, nylon, thermoplastic polyurethane; biodegradable synthetic material, e.g., polyglycolide polylactides, poly(caprolactone), poly(dioxanone), poly(ethylene glycol)diacrylate, poly(N-isopropylacrylamide); biomaterial, e.g., gelatine, chitosan, alginate, agarose, hyaluronic acid derivatives, fibrin glue, elastin, cellulose, methylcellulose, fibronectin, collagen, silk; hydrogel; ionic gel; liquid crystal polymer, elastomer or gel; shape memory polymer; photoresist polymer, e.g., SU-8; biological protein, e.g., squid ring teeth protein; fabric material; non-magnetic metal; silicon; silica; glass; wood; carbon fibre; and derivates and combinations of the foregoing. Such materials can be used to provide a soft device in comparison to a rigid device.

The magnetic elements may be selected from the group of members consisting of chromium dioxide ($CrO_2$), samarium-cobalt (SmCo), neodymium-Iron-Boron (NdFeB), cobalt (Co), ferrite, permalloy (NiFe), carbon steel, tungsten steel, Alnico, iron, stainless steel, nickel (Ni), iron platinum (FePt), iron oxide ($Fe_2O_3$), barium ferrite, magnetite; combinations, alloys or composites of the foregoing. Such materials can be programmed and reprogrammed by heating to or above their Curie temperature.

In accordance with a further aspect the present invention relates to a method of encoding a programmable and/or reprogrammable magnetic soft device as discussed herein, the method comprising the steps of:

- heating the composite material to a temperature selected in the range of 25% below the Curie temperature to a temperature 25 % above the Curie temperature measured in °C of the magnetic elements distributed therein;
- cooling the composite material; and
- re-orienting magnetic domains of the magnetic elements by applying an external magnetic field during cooling, or during both heating and cooling.

The magnetic elements, such as particles, only realign themselves after the Curie temperature point has been achieved. Once the Curie temperature point has been reached and the magnetic elements have realigned themselves an external magnetic field can be used to realign, i.e. program the magnetic elements, i.e. particles, in order to have the desired new alignment, i.e. programming of the magnetic domains.

The magnetic field may also be applied during the step of heating, but in any event has to be applied during the step of cooling in order to be a able to re-orient the magnetic domains of the magnetic elements.

The steps of heating and cooling the composite may be carried out sequentially by sequentially focusing a tunable light source, i.e. a collimated laser, onto regions of said composite and cooling said regions optionally before moving on to further regions of said composite. In this way each region of the device can be programmed with its own magnetization profile.

The steps of heating and cooling the composite may be carried out globally by using a convection oven of said composite and cooling the composite with a magnetic master placed adjacent to the said composite. Such an assembly can be effectively used for batch processing of identical types of devices.

The step of reprogramming can also be achieved by using a magnetic master, e.g. comprising a jig and a magnetic field generating device, or simply a magnetic field generating device providing a magnetic field of pre-defined orientation and magnitude, and globally heating everything in one-shot. Magnetic masters can be configured to generate arbitrary magnetization fields in neighboring sections and placed nearby the sample to be magnetized. Then heating the all sections of the sample will allow magnetization. The magnetic master should possess a Curie temperature above the applied heating in this process.

The step of applying the magnetic field may be carried out with a magnetic field having a magnitude selected in the range of 1 mT to 10 T, in particular in the range of 15 mT to 3 T, in particular for each cooling cycle to orient each region of the composite with its own magnetic magnetization profile.

This magnetization profile may differ from or be the same as a magnetization profile of other regions of the composite. In this way one can program different parts of the device with different magnetization profiles in such a way that each part of the device can carry out one or more specific movement types.

A further aspect which is not part of the present invention relates to a use of a programmable and/or reprogrammable magnetic soft device manufactured and/or to an untethered programmable and/or reprogrammable 3D magnetic soft device as at least one of a reconfigurable gripper, a programmable and/or reprogrammable acoustic wave guide, a programmable and/or reprogrammable electronic circuit, a programmable and/or reprogrammable antenna, programmable and/or reprogrammable mechanical metamaterials, programmable and/or reprogrammable wearable pieces of equipment, adaptive medical robots and combinations of the foregoing.

The present invention will be described in detail with reference to the following drawings. There is shown:

Fig. 1a to i    steps carried out for heat-assisted 3D magnetic programming and reprogramming of magnetic active soft matter at an untethered programmable and/or reprogrammable 3D magnetic soft device, and sample devices;

EP 4 118 666 B1

Fig. 2a to p — various magnetic soft devices that are reprogrammable and their responses to the application to external magnetic fields after initial programming and after reprogramming;

Fig. 3a to h — various kinds of structures of magnetic soft device before and after reprogramming their magnetization;

Fig. 4a to j — examples of heat-assisted magnetic programming of magnetic soft matter at the microscale;

Fig. 5a to c — mechanical properties of the magnetic soft elastomer;

Fig. 6a to d — magnetic properties of the device;

Fig. 7a to c — photothermal response of the magnetic soft elastomer forming part of the composite;

Fig. 8a to d — discrete and 3D magnetization of magnetic active soft matter forming part of the composite;

Fig. 9a to g — stacked two half-sphere structures forming the device, transforming into a full sphere upon magnetic actuation;

Fig. 10a to j — reprogrammable magnetization of devices;

Fig. 11a to b — reprogrammable magnetization of flexible magnetic leaves as devices;

Fig. 12 — a performance comparison of previous magnetic soft material programming approaches with the current work;

Fig. 13a to f — computational modeling and validation of shape deformations of devices;

Fig. 14a — heat-assisted magnetization and magnetic actuation apparatus; and

Fig. 15a to b — photomask design and critical dimensions employed in micropatterned laser heating (a) and fabrication of polyurethane NdFeB magnet for contact transfer of magnetic profiles (b).

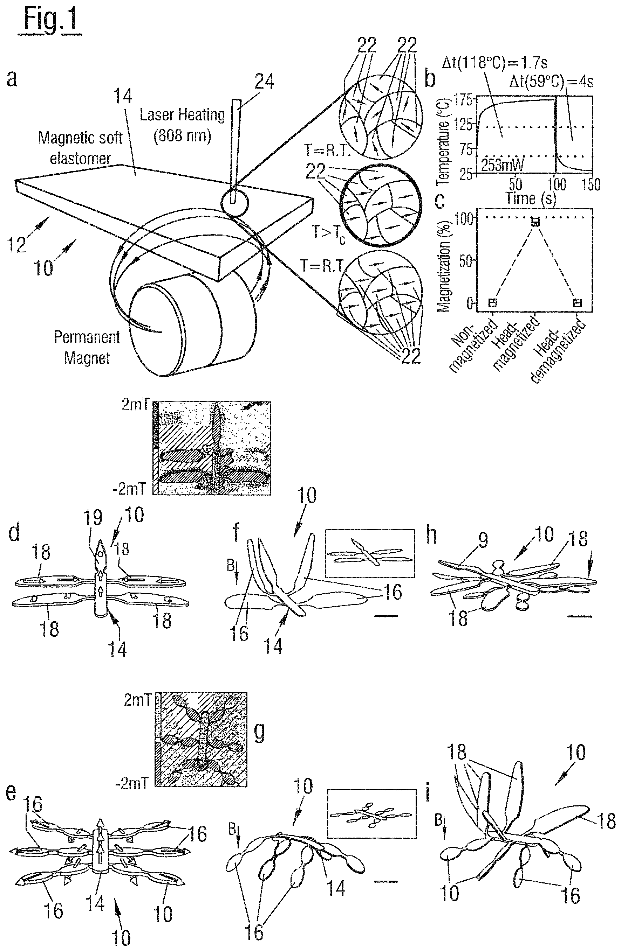

Fig. 1 shows the steps carried out for heat-assisted 3D magnetic programming and reprogramming of magnetic active soft matter at an untethered programmable and/or reprogrammable 3D magnetic soft device 10. The 3D magnetic soft device 10 has one or more parts and/or regions with Young's modulus of less than 500 MPa. The programmable and/or reprogrammable 3D magnetic soft device 10 comprises a body 14 formed of a composite 12. In Fig. 1a the body has the shape of a bar, whereas in Fig. 1d to 1i, the body 14 generally has the shape of a dragonfly with parts 16 in the form of several legs 16, wings 18, and a tail 19 extending from the body 14. Additionally, in Fig. 1h and 1i, the body has parts in the form of legs 16 and wings 18 extending therefrom.

The legs 16, wings 18 and tail 19 can be formed of the same composite 12 as the body 14 or of further composites 12, differing in their material composition and/or material properties, such as hardness, stiffness, magnetization profile etc. Parts or regions of the body 14 may comprise material without magnetic elements embedded therein. The respective composite 12 having a magnetization profile which is non-zero comprises a base material and magnetic elements distributed within said base material.

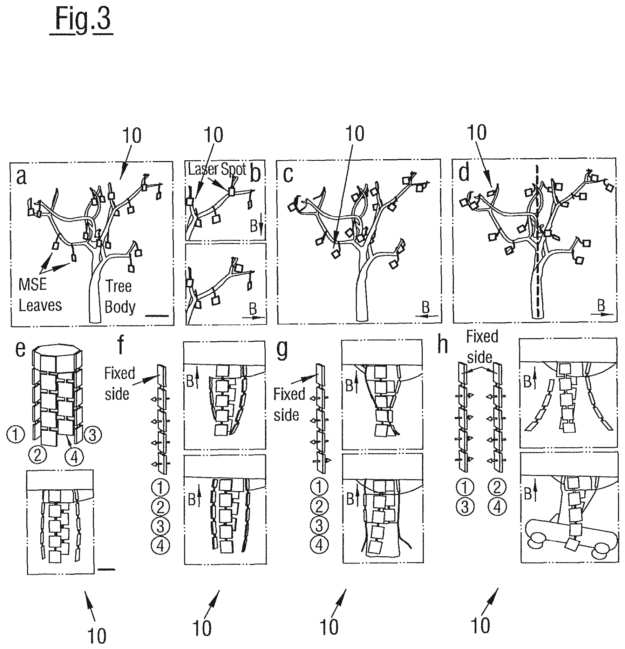

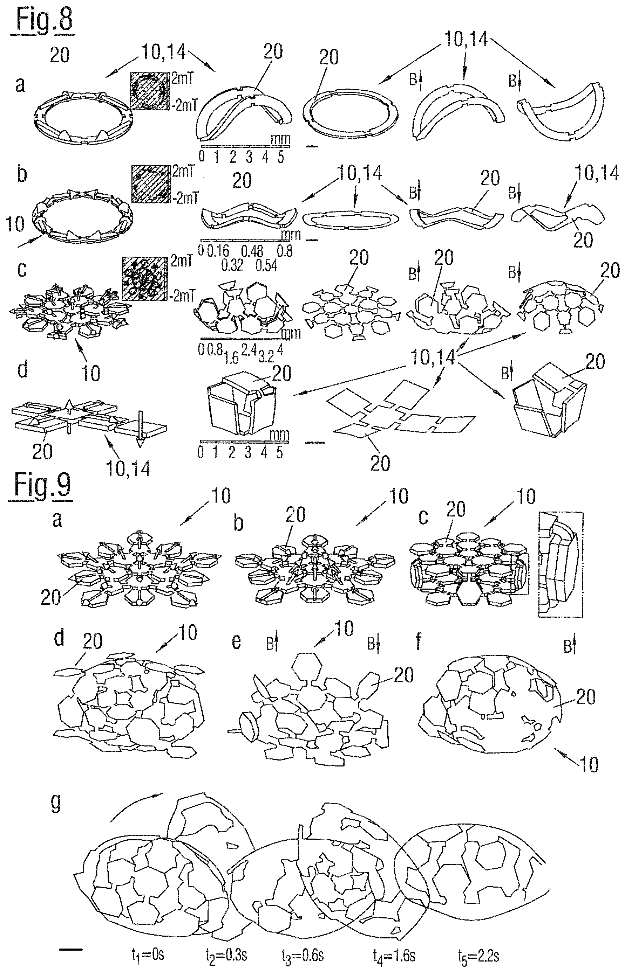

The body 14, the legs 16, the wings 18, the tail 19 and any further shapes or sections 20 (see e.g. Fig. 8) or fingers 34 (see Fig. 3e to h) of the 3D magnetic soft device 10 have an arbitrary magnetization profile, with different regions of the 3D magnetic soft device 10, i.e. the body 14, the legs 16, the wings 18, the tail 19, the sections 20, and/or the fingers 34, having different magnetization profiles. The information encoded into the programmable and/or reprogrammable 3D magnetic soft device 10 comprises shape changing instructions for changing a shape of at least some of the regions of the 3D magnetic soft device 10, i.e. the body 14, the legs 16, the wings 18, the tail 19 and the sections 20, relative to one another on application of an external field.

In this connection it should be noted that the base material used to form the various parts, i.e. the body 14, the legs 16, the wings 18, the tail 19, the sections 20, the fingers 34 etc., of the programmable and/or reprogrammable 3D magnetic soft device 10 may be selected from the group of members consisting of elastomers, thermoplastic elastomers, rubbers, duroplastics, thermoplastics, e.g., polydimethylsiloxane, aliphatic aromatic copolyester or modified polyester, or modified copolyester, polyurethane elastomer, silicone rubber, natural rubber, latex, styrene ethylene butylene styrene, butyl rubber, fluorosilicone rubber, polyester, nylon, thermoplastic polyurethane; biodegradable synthetic material, e.g., polyglycolide polylactides, poly(caprolactone), poly(dioxanone), poly(ethylene glycol)diacrylate, poly(N-isopropylacrylamide); biomaterial, e.g., gelatine, chitosan, alginate, agarose, hyaluronic acid derivatives, fibrin glue, elastin, cellulose, methylcellulose, fibronectin, collagen, silk; hydrogel; ionic gel; liquid crystal polymer, elastomer or gel; shape memory polymer; photoresist polymer, e.g., SU-8; biological protein, e.g., squid ring teeth protein; fabric material; non-magnetic metal; silicon; silica; glass; wood; carbon fibre; and derivates and combinations of the foregoing.

In this connection it should be noted that the magnetic elements used in the various parts, i.e. the body 14, the legs 16, the wings 18, the tail 19, the sections 20, and the fingers 34 etc., of the programmable and/or reprogrammable 3D magnetic soft device 10 may be selected from the group of members consisting of chromium dioxide, samarium-cobalt, neodymium-Iron-Boron, cobalt, ferrite, permalloy, carbon steel, tungsten steel, Alnico, iron, stainless steel, nickel, iron platinum, iron oxide, barium ferrite, magnetite; combinations, alloys or composites of the foregoing.

In this connection it should further be noted that the magnetic elements may be present in the form of particles, rods, wires, disks, spheroids, whiskers, irregular particles, Janus particles, and combinations of the foregoing.

In the example of Fig. 1a, the body 14 having the bar shape of the 3D magnetic soft device 10 is formed of a magnetic soft elastomer, composed of magnetic $CrO_2$ particles embedded in polydimethylsiloxane (PDMS). In order to form the 3D magnetic soft device 10 the following steps are carried out:

- the composite 12 of base material and magnetic elements distributed within said base material is formed, e.g. by simply mixing the magnetic elements, e.g. the magnetic $CrO_2$ particles, with a relatively soft material, such as the PDMS;
- the composite 12 is then shaped to have a desired final shape. In the example of Fig. 1a the desired shape is the shape of a bar which can be formed by e.g. pouring the not yet solidified composite material into a mold and solidifying it there. Other forms of manufacture of the complete 3D magnetic soft device 10 may comprise 3D printing of parts, injection molding of parts, cutting parts from bulk material etc.;
- once at least a part, i.e. the body 14, the legs 16, the wings 18, the tail 19, sections 20 and/or fingers 34 of the 3D magnetic soft device 10 have been shaped, either the parts on their own, or the complete 3D magnetic soft device 10 is heated. During the step of heating the composite 12, a magnetic field may be applied at the composite 12 or not; and
- thereafter the composite 12 is cooled while applying a magnetic field at the composite 12, with the step of heating 12 comprising heating the composite 12 to a temperature close to or above the Curie temperature of said magnetic elements, i.e. to a temperature which is in the range of 10% below the Curie temperature, preferably to a temperature at most 10° C, in particular 5° C, below the Curie temperature if $CrO_2$ is the magnetic element and at most 5° C below a melting point of the base material in order to prevent the base material from being molten.

In this connection it should be noted that the step of heating may be carried out before, after and/or during the step of shaping the composite.

In this connection it should further be noted that the step of shaping and the step of heating may be carried out simultaneously.

In this connection it should further be noted that the magnetic field applied during the heating and/or cooling step is below the coercive magnetic field of the magnetic element at its room temperature state, i.e. generally speaking between 25 to 99 % below the coercive magnetic field of the magnetic element. For e.g. $CrO_2$ the magnetic field applied during the heating and/or cooling step may be between 50 to 95% below the coercive magnetic field of the magnetic element.

The step of shaping the composite 12 may comprise at least one of the following steps; molding the composite 12 in one mold of pre-defined shape and size, molding one or more parts of the composite 12 in one or more molds of same shape and size, molding the composite 12 in one or more molds of differing shapes and sizes, 3D printing the composite 12, 3D printing one or more parts of the composite 12, combining parts of the composite 12, cutting sections of material from the composite 12, cutting sections of material from parts of the composite 12 and combinations of the foregoing.

The melting temperature of the base material may be higher than the maximum temperature applied to the magnetic composite 12 during the heating step in order to prevent a phase change of the base material.

The steps of heating and cooling the composite 12 may be carried out a plurality of times sequentially for different regions 22 of the composite 12. Alternatively, the steps of heating and cooling the composite 12 may be carried out once for the complete 3D magnetic soft device 10.

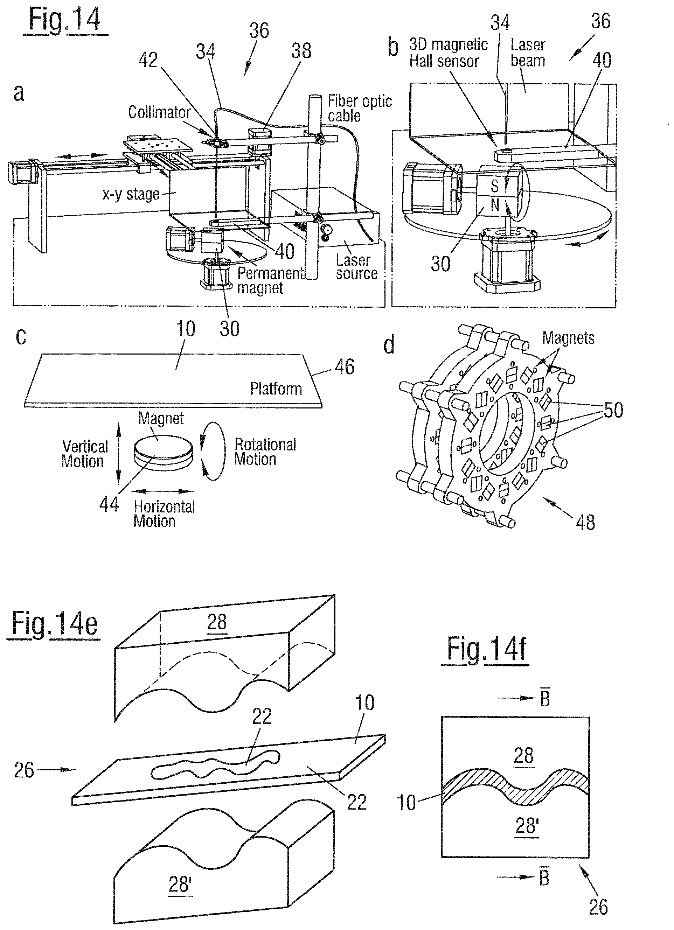

The step of magnetization may be carried out for each step of cooling for each region 22 of the composite 12 so that each region 22 is provided with its own magnetization direction. Alternatively, the step of magnetization of the composite 12 may be carried out once for the complete 3D magnetic soft device 10, if. e.g. a Jig 26 (see Fig. 14e and Fig. 14f) or the like are used to predefine a movement shape of the complete 3D magnetic soft device 10.

As indicated in Fig. 1a, the step of heating the composite 12 can be carried out with a tunable laser 24. This is done to locally heat different regions 22 (see the extracts of Fig. 1a) and to then locally apply a magnetic field to program the current region 22 of interest with its own magnetization profile. As indicated in the various extracts the different regions can be magnetized with varying magnetizations profiles or with the same or similar magnetization profiles (e.g. comprising direction of magnetization and magnitude of magnetization).

In this connection it should be noted that an average diameter respectively width of each region 22 can be selected in the range of 1 $\mu$m to 100 mm, in particular in the range of 20 $\mu$m to 50 mm, in dependence on the heating device and e.g. its optical components used to bring about a heating of the specific region 22.

The step of applying the magnetic field may be carried out with a magnetic field having a magnitude selected in the range of 1 mT to 10 T. In the example of Fig. 1a the magnetic field is applied using a permanent magnet 30, other sources of magnetic field may also be used in order to magnetize the regions 22 of the 3D magnetic soft device 10.

Once the regions 22 are locally heated to close to and preferably above the Curie temperature of the particles via the laser 24, the magnetic elements, e.g. the particles, lose their permanent magnetization and their magnetization direction is reoriented by applying the external magnetic field during the step of cooling.

Fig. 1b shows the behavior of heating and cooling curves for the magnetic soft elastomer, i.e. the composite 12, when the composite 12 is heated above the Curie temperature of the $CrO_2$ particles (118 °C) in 1.7 s and cooled down to half of this temperature in 4 s.

Fig. 1c shows that the magnetic soft elastomer, i.e. the composite 12, is magnetized with 90% efficiency by heat-assisted magnetization and demagnetized by only heating above the Curie temperature without any external magnetic field, with error bars representing the standard deviation of the mean.

Figs. 1d to 1g show examples of the 3D magnetic soft device 10 cut into the shapes of a body with a tail 19 and wings 18 (Fig. 1d) and a six-legged 16 body (Fig. 1e) with corresponding magnetization directions (indicated by arrows) and out-of-plane magnetic flux profile measurements. The insets indicate magnetic flux density strength and the scale bars are 2 mm.

Figs. 1f and 1g show that upon magnetic actuation of the 3D magnetic soft device 10 of Figs. 1d and 1e, individual parts 16, 18, 19 (the legs 16, the wings 18 and the tail 19) underwent shape-change in accordance with their programmed magnetization directions, with the insets showing initial shape of the structures in the absence of magnetic fields.

Fig. 1h and 1i show 3D magnetic soft devices 10 with wings 18 and legs 16 that are stacked to generate a 3D hierarchical "dragonfly" structure upon magnetic actuation. The scale bar is 2 mm. Actuation of the 3D magnetic soft devices 10 is performed by applying magnetic fields of 60 mT in the directions indicated with the arrows.

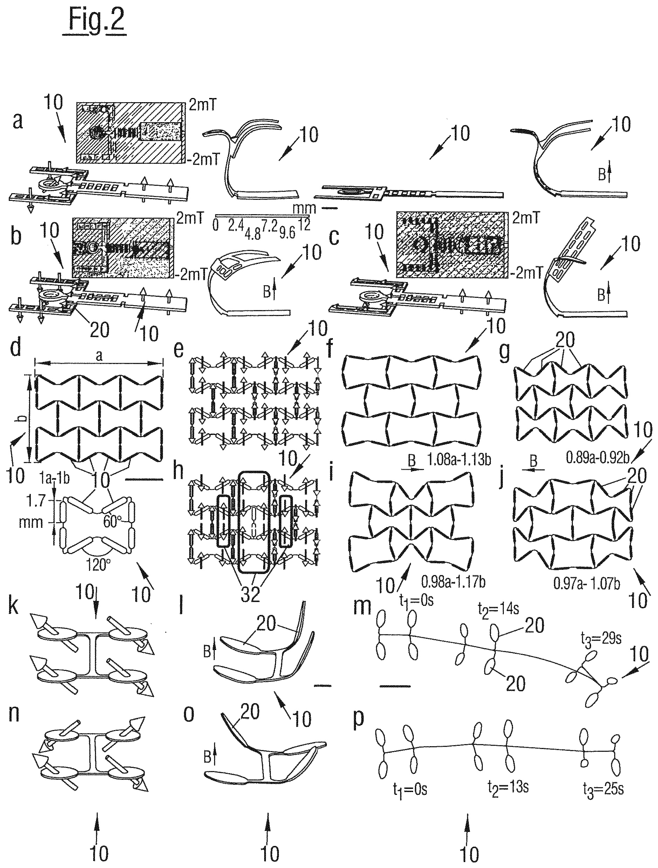

Fig. 2a shows distributed 3D magnetizations, out-of-plane magnetic flux density profile measurements, finite element simulations, and experimental shape-changes upon magnetic actuation of a "stickman" structure.

Figs. 2b and 2c show how reprogramming the distributed magnetization directions of the magnetic soft device 10 of Fig. 2a in the form of a stickman structure enables reconfiguring the shape-changes such that the stickman structure 10 can change its shape in a different manner after reprogramming. The arrows indicate local magnetization directions. Also indicated are magnetic flux density strength and total deformation, respectively, the scale bar is 1 mm.

Fig. 2d to 2j show how the mechanical behavior of an auxetic metamaterial can be tuned by reprogramming distributed magnetization profile of individual units, i.e. devices 10. Length and width of the whole structure 10 is denoted with a and b, respectively.

Fig. 2e to 2g show that the flexible auxetic structure expands and compresses both in length and width depending on magnetic actuation direction, displaying that the device 10 has a negative Poisson's ratio.

Fig. 2h to 2j show how the reprogramming of the magnetization profile of three units in the middle results in expansion in width with minimal change in length irrespective of magnetic actuation direction. The arrows indicate local magnetization directions. Boxes 32 show the reprogrammed regions 22 of the magnetic soft device 10, the scale bar is 5 mm. Actuation of the structures is performed by applying uniform magnetic fields of 60 mT in the directions indicated with the arrows beneath the B denoting the magnetic field.

Fig. 2k to 2p show how reprogramming the magnetization profile of a 4-legged 16 flexible robot 10 enables tunable locomotion patterns. Fig. 2k and 2n show how the magnetization directions of the legs 16 are programmed to generate different deformation configurations, with the arrows passing through the legs 16 indicating local magnetization directions. Figs. 2l and 2o show that upon magnetic actuation (20 mT), indicated with the arrows beneath the B, the legs 16 deform in accordance with their magnetization directions, the scale bar is 1 mm.

Figs. 2m and 2 p show flexible robots, i.e. devices 10, with different magnetization profiles generating different locomotion patterns with rotational magnetic actuation. Scale bar is 5 mm.

Figs. 3a to 3d show the reprogrammable magnetization of flexible magnetic leaves as magnetic soft devices 10 distributed on a 3D-printed non-magnetic body.

Fig. 3b and 3c show how the magnetization direction of individual leaves is programmed by local laser-heating using the laser 24. Fig. 3d shows that the magnetization direction of half of the leaves, indicated by the dashed line, is reprogrammed in the reverse direction, with the scale bar being 5 mm. Fig. 3e to 3h shows a 4-finger 34 adaptive soft gripper, as a magnetic soft device 10, enabled by reprogramming magnetization profile of the fingers.

Figs. 3f to 3h show shape-deformation of the fingers 34 are reprogrammed to grasp objects with different morphologies, including a sphere (3 mm diameter) (see Fig. 3f), a rod (4 mm diameter and 4 mm height) (see Fig. 3g), and a car representing complex morphology (10.6 mm length, 3 mm width, and 1.75 mm height) (see Fig. 3h). The arrows indicate local magnetization directions and the scale bar is 2 mm. Actuation of the structures is performed by applying uniform magnetic fields of 60 mT in the directions indicated with the arrows beneath the B.

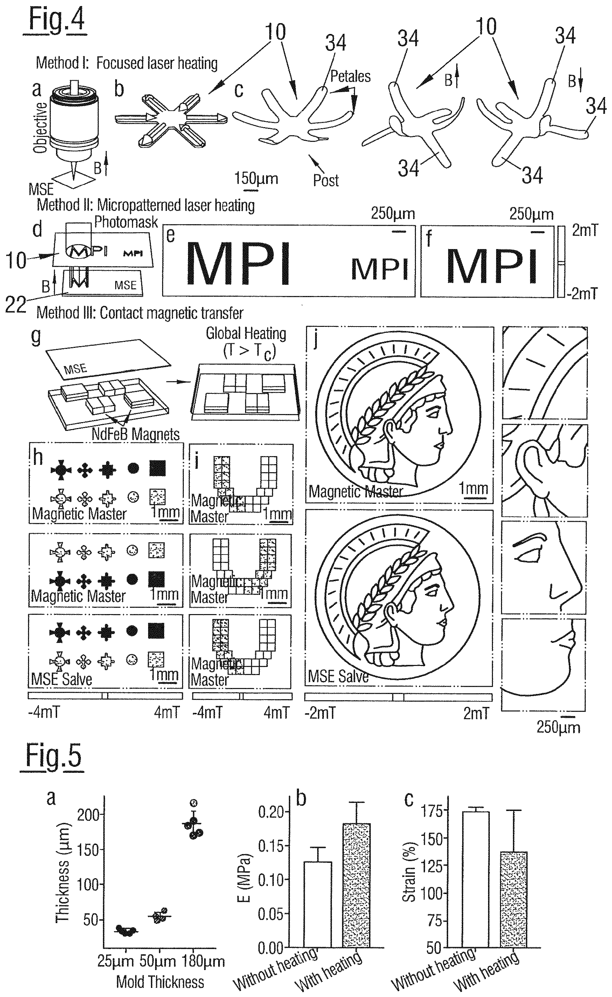

Fig. 4a shows scanning a focused laser spot from the laser 24 on a region 22 of the magnetic soft elastomer (MSE) as a magnetic soft device 10, which creates a precisely controlled local heating of the desired region 22. Scanning the device 10 with the laser in a desired pattern is used to program the magnetization profile on that material.

Fig. 4b and 4c show and an example soft structure, i.e. a magnetic soft device 10 with 6 petals (fingers 34) (150 $\mu$m width, 500 $\mu$m length, and 30 $\mu$m thickness) is placed on a micropost. Red arrows indicate the magnetization directions of the petals. Magnetic actuation (60 mT) resulted in deformation of petals in reverse directions. Fig. 4d indicates how a collimated laser 34 can heat a desired shape on a target magnetic soft elastomer, i.e. region 22, in one shot through a mask containing the micropattern of such desired shape.

Figs. 4e and 4f show magnetic flux density measurements of example magnetically programmed samples 10 by such micropatterned laser-heating. The smallest magnetic pattern is 80 $\mu$m in width and the scale bars are 250 $\mu$m.

Fig. 4g shows contact transfer of desired magnetic profiles in one shot via global heating. The magnetic soft elastomer, i.e. the composite 12, is placed in direct contact with NdFeB magnets, with a greater Curie temperature, arranged in different configurations and heated above the Curie temperature of the $CrO_2$. Magnetization directions of the NdFeB magnets are transferred to the magnetic soft elastomer during cooling.

Fig. 4h and 4i show magnetic flux density measurements of the NdFeB masters of different example shapes and configurations and the magnetic soft elastomer slaves, with the scale bars being 500 $\mu$m and 1 mm, respectively. Fig. 4j shows the contact transfer of a complex magnetization profile in the geometric pattern of "Minerva". Insets show close up views of the magnetic flux density profile of the magnetic soft elastomer slave. Smallest magnetic pattern is 38 $\mu$m in width. The bars indicate magnetic flux density strength, with the scale bars being 1 mm and 250 $\mu$m, respectively.

Fig. 5a shows how a film thickness of the fabricated magnetic soft elastomers forming part of the composite 12 of the device 10 is controlled by changing the mold depth between 25 $\mu$m to 200 $\mu$m.

Figs. 5b and 5c show the elastic modulus (E) and tensile strain of the magnetic soft elastomers forming part of the composite 12 of the device 10 before and after laser-heating above 150 °C. Error bars represent the standard deviation of the mean.

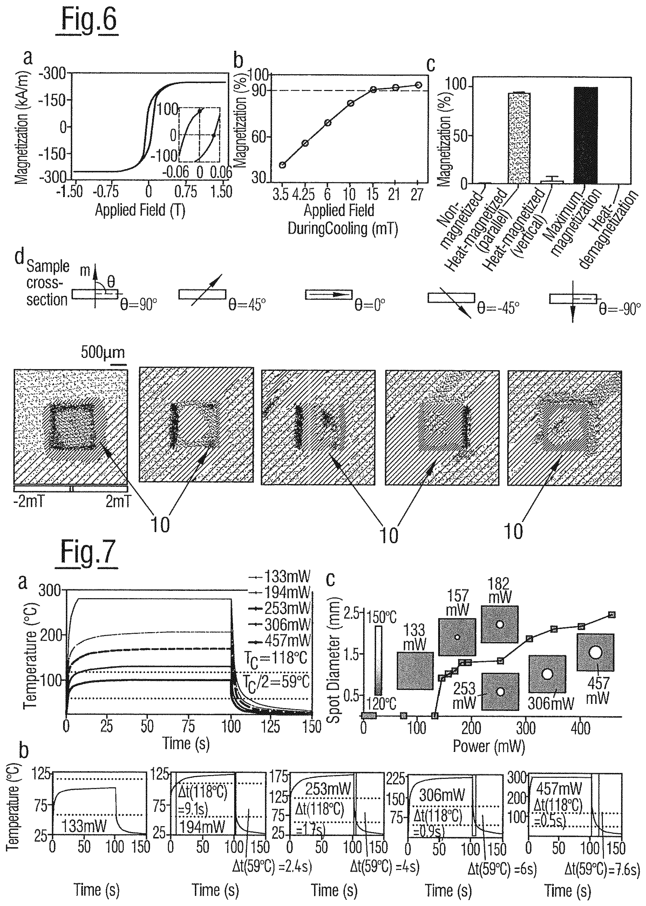

Fig. 6a shows a hysteresis loop of the $CrO_2$ at room temperature. The remaining magnetization ($M_r$) of $CrO_2$ particles is 109 kA/m and their coercivity is 67 mT. Fig. 6b shows the effect of applied magnetic field strength during cooling on magnetization efficiency and Fig. 6c shows the heat-assisted magnetization efficiency in directions parallel and vertical to the magnetization direction and heat-assisted demagnetization. Magnetization values for samples magnetized under uniform 1.8 T field were considered as 100%. Error bars represent the standard deviation of the mean. Fig. 6d shows measured out-of-plane magnetic flux density profiles of a rectangular magnetic soft elastomer sample magnetized in varying directions.

Fig. 7a shows the effect of laser power on average temperature of the magnetic soft elastomers forming part of the composite 12 of the device 10. Fig. 7b shows heating-cooling durations of the magnetic soft elastomers forming part of the composite 12 of the device 10. Fig. 7c shows the effect of laser power on spot diameter heated above the Curie temperature of $CrO_2$.

Fig. 8a to 8d show distributed 3D magnetizations, out-of-plane magnetic flux density profile measurements, finite element simulations, and experimental shape-changes upon magnetic actuation of a 4-segment ring (Fig. 8a), 8-segment ring (Fig. 8b) a half-sphere (Fig. 8c), and a cube structure (Fig. 8d). The scale bars are 1 mm, and the actuation was performed by applying a magnetic field of 60 mT in the directions indicated with the black arrows.

Fig. 9a and 9b show magnetization directions of sections 20 of the two half-sphere structures forming the device 10. Fig. 9c shows the manual assembly of the two-half ball structures realized by sticking together the corresponding hexagonal parts at the periphery, as shown in the inset to form the device 10. Fig. 9d to 9f show an out-of-plane compression and formation of a closed spherical structure upon magnetic actuation (60 mT) in the directions indicated with the arrows beneath the B. Fig. 9g show rotational magnetic actuation results in rolling of the spherical structure while preserving its closed formation. The scale bars are 1 mm.

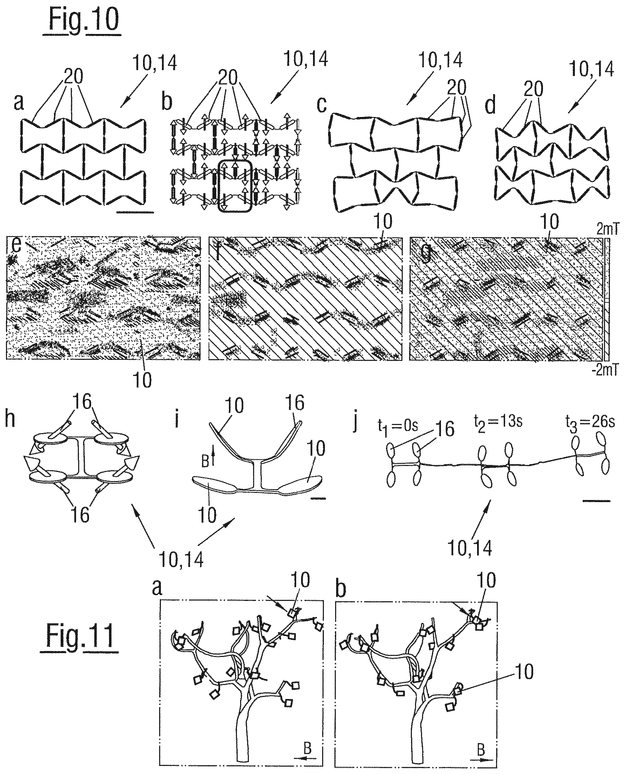

Figs. 10a to 10d show how reprogramming the magnetization profile of a single device 10 in the auxetic metamaterial results in heterogeneous deformation within the structure. The arrows indicate local magnetization directions. The box 32 with the solid lines show the reprogrammed regions 22. Scale bar is 5 mm.

Fig. 10e to 10g show out-of-plane magnetic flux density profile measurements of the auxetic metamaterials shown in Fig. 2e (e), Fig. 10b (f), and Fig. 2h (g). The bars indicate magnetic flux density strength.

Fig. 10h to 10j show the reprogrammed magnetization profile of a 4-legged flexible robot enables tunable locomotion pattern, with Fig. 10h and 10i showing how the legs deform in accordance with their magnetization directions upon magnetic actuation (20 mT) indicated with the arrows. The scale bar is 1 mm. Fig. 10j shows the locomotion of a 4-legged flexible robot 10 in a linear trajectory under rotational magnetic field actuation. The scale bar is 5 mm.

Fig. 11a and 11b show the reprogrammable magnetization of flexible magnetic leaves as devices 10. Magnetization direction of two leaves (as shown with the arrows) are reprogrammed in the opposite direction of the remaining leaves. Fig. 10a and 10b show how the magnetic actuation (60 mT in the directions indicated with the arrows beneath the B) resulted in their reverse deformation compared to the other leaves. The scale bar is 5 mm.

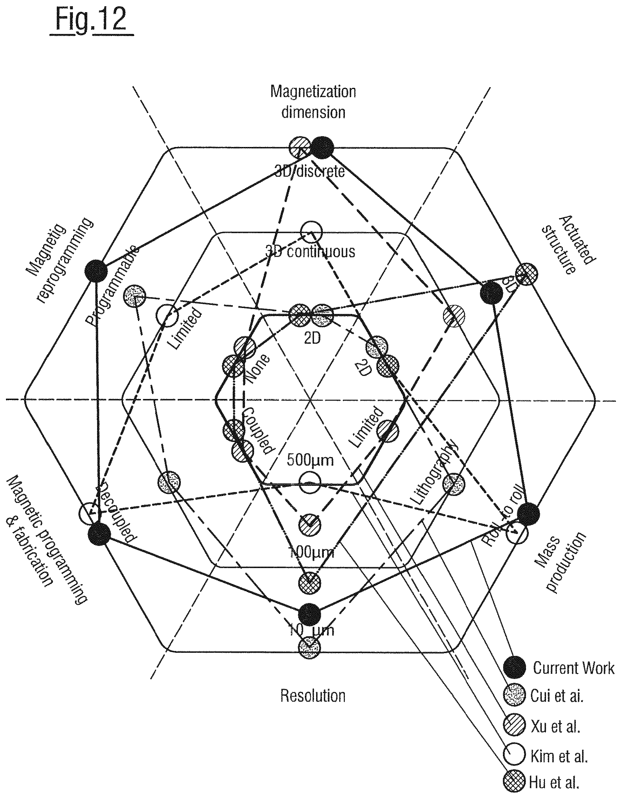

Fig. 12 shows a performance comparison of previous magnetic soft material programming approaches with the current study. The results of the previous studies can be found in the following publications Hu, W., Lum, G. Z., Mastrangeli, M. & Sitti, M. Small-scale soft-bodied robot with multimodal locomotion. Nature 554, 81-85, (2018), Xu, T., Zhang, J., Salehizadeh, M., Onaizah, O. & Diller, E. Millimeter-scale flexible robots with programmable three-dimensional magnetization and motions. Science Robotics 4, eaav4494, (2019), Cui, J. et al. Nanomagnetic encoding of shape-morphing micromachines. Nature 575, 164-168, (2019), and Kim, Y., Yuk, H., Zhao, R., Chester, S. A. & Zhao, X. Printing ferromagnetic domains for untethered fast-transforming soft materials. Nature 558, 274-279, (2018).

Magnetization and related fabrication capabilities of the heat-assisted magnetic programming strategy presented herein are compared with those of existing magnetic programming approaches for soft materials in the literature. Magnetization dimension indicates the degree of freedom available for magnetization, where 3D refers to the capability to magnetize in arbitrary direction. In continuous magnetization, neighboring sections cannot have sharp changes in magnetization, whereas discrete magnetization enable independent magnetization of adjacent sections. In reprogrammability, limited refers to reprogramming in directions designated during fabrication and technically challenging approaches at small scale. Actuated structure refers to the dimension of soft systems demonstrated in different approaches. In magnetic programming and fabrication, coupled refers to magnetic programming during the fabrication process and decoupled indicates magnetic programming afterwards the fabrication. In mass production, limited refers to restricted high-throughput production capability compared to lithography and roll to roll compatible methods.

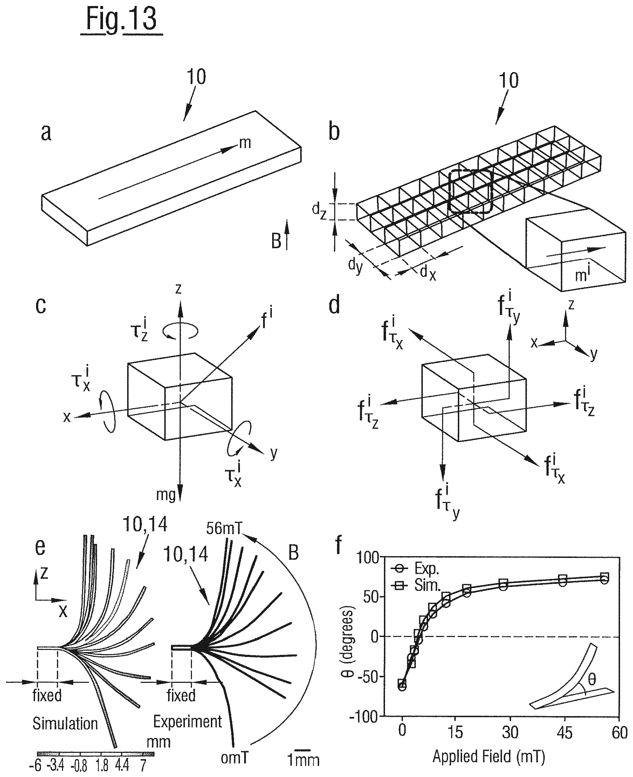

Fig. 13a shows a sample beam structure with a net magnetic moment m. Fig. 13b shows the beam structures is divided into smaller sub-sections labeled by '/' with the pre-defined lengths of $d_x$, $d_y$ and $d_z$, and net magnetic moment $\boldsymbol{m^i}$.

Fig. 13c shows a free body diagram of a subsection with $\boldsymbol{f^i}$, $\tau_x^i$, $\tau_y^i$, $\tau_z^i$ and $mg$ representing magnetic force, magnetic torques on Cartesian axes, and gravitational force, respectively.

Fig. 13d shows a distribution of the magnetic torques $\tau_x^i$, $\tau_y^i$, and $\tau_z^i$ as the boundary loads $f_{\tau_x}^i$, $f_{\tau_y}^i$, and $f_{\tau_z}^i$ on the subsection facets. Fig. 13e shows simulation and experiment results for a beam (10 mm length x 1 mm width x 0.17 mm thickness) with a magnetization profile vertical to the applied external magnetic field. Fig. 13f shows deflection angle ($\theta$) values obtained from simulations and experiments depending on the applied external magnetic field.

Fig. 14a and 14b show how a magnetization setup 36 consists of a motorized stage 38, a NdFeB permanent magnet 30 that can rotate in 360°, a 3D magnetic hall-effect sensor 40, and a power-adjustable fiber-coupled NIR laser 34 with a collimator 42. (c) For magnetic actuation, a disc shaped magnet 44 (60 mm diameter and 10 mm thick) was moved in vertical or horizontal directions, or rotated underneath an actuation platform 46. Fig. 14d shows a Halbach array 48, composed of 16 permanent magnets 50 (10 mm x10 mm x 10 mm) arranged as shown, was used for generation of the uniform magnetic field for magnetic actuation.

Fig. 14e and 14f show a Jig 26 that can be used to program the device 10, for example a bar shaped device 10. The Jig comprises top and bottom halves 28, 28' that are clamped around the device 10 to be programmed and/or reprogrammed. Once the device 10 is heated to close to or above the Curie temperature of the magnetic elements of the device 10, a magnetic field B can be applied as indicated in Fig. 14e to magnetize the different regions 22 of the device 10 with different orientations of magnetization, with the different orientations being dependent on the shape of the internal structures of the Jig 26.

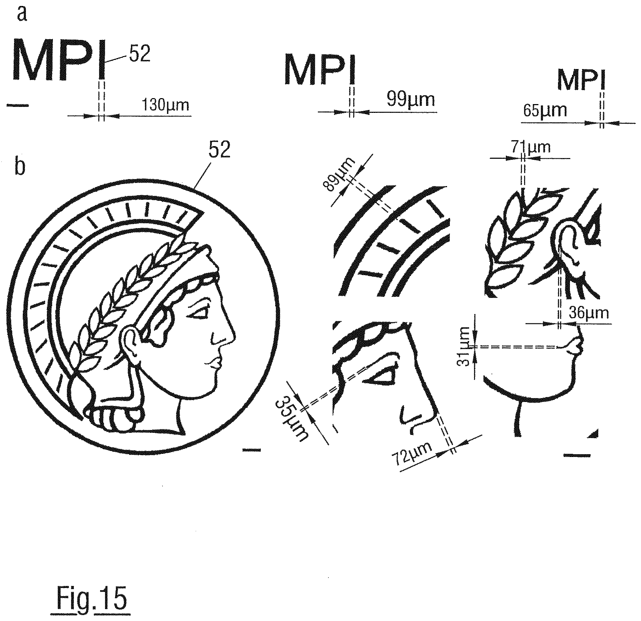

Fig. 15a and 15b show various photomask 52 designs and critical dimensions employed in micropatterned laser heating (see Fig. 15a) and fabrication of polyurethane NdFeB magnet for contact transfer of magnetic profiles (see Fig. 15b). The scale bars are 500 $\mu$m.

The devices 10 shown in the foregoing disclose devices 10 of programmable magnetic soft matter, in which magnetic micro/nanoparticles are embedded in soft polymers. Such devices 10 are promising for the development of untethered (wireless) devices or robots with complex deformation and locomotion capabilities that can operate at small scales. Magnetic fields generate torque on magnetic soft materials until the magnetization direction of all domains are aligned with the applied field direction. Therefore, creating a spatial distribution of magnetization directions in a magnetic soft material enables programmable shape-deformation under magnetic fields. Current three-dimensional (3D), discrete magnetic programming approaches rely on arranging physical orientation of ferromagnetic particles or alignment of superparamagnetic particles in polymer matrices during curing, which prevents reprogramming once fabricated. In this work, we use heat-assisted magnetic programming of soft materials by heating above the Curie temperature of the ferromagnetic particles and reorienting their magnetic domains with external magnetic fields during cooling (Fig. 1a). By sequential heat-assisted magnetization over a magnetic soft body, shape-changing instructions in 3D can be encoded discretely and reprogrammed on demand.

The presented magnetic soft elastomers are composed of chromium dioxide ($CrO_2$) microparticles with an average diameter of 10 $\mu$m embedded in a polydimethylsiloxane (PDMS) elastomer. $CrO_2$ is a ferromagnetic material (Fig. 6a), which has a Curie temperature of 118 °C, enabling heat-assisted magnetic (re)programming within the operation temperature of most elastomers.

The $CrO_2$/PDMS magnetic soft elastomer composite sheets are prepared by curing the $CrO_2$ particles and PDMS mixture in molds of different thicknesses, resulting in magneto-elastic films in a range of 25-200 $\mu$m thickness (Fig. 5a).

In this connection it should be noted that the devices 10 can have device thicknesses of at least some of their parts selected in the range of 25-200 $\mu$m thickness. It is also possible that the devices 10 can have device thicknesses of at least some of their parts selected in the range of 10 $\mu$m to 10 mm thickness.

A collimated near-infrared (NIR) laser with tunable power is used for heating the magnetic soft elastomers locally and precisely with controlled temperature, heating-cooling duration, and heated spot size (Fig. 1b and Fig. 7). Shortest heating-cooling cycle in a 1.3 mm-diameter heated spot is achieved in 5.7 s (Fig. 1b). The laser-heated magnetic soft elastomer spots are magnetized via external magnetic fields exceeding 15 mT, resulting in more than 90% magnetization efficiency in comparison to maximum achievable magnetization under a 1.8 T magnetic field (Fig. 5b).

Such high magnetization efficiencies indicate almost complete reorientation of magnetic domains in the desired direction, while minimizing undesired magnetization in other directions. The same materials can be then demagnetized locally or fully by heating again above the Curie temperature of $CrO_2$ particles in the absence of a magnetic field (Fig. 1c and Fig. 5c). Momentary heating has limited effect on the mechanical properties of the magnetic soft elastomers (Fig. 6b, c), since the applied temperatures are well within the curing and operation temperature range of PDMS, enabling non-invasive magnetic programming and reprogramming.

To illustrate heat-assisted magnetic programming of $CrO_2$ particle-embedded soft materials, planar magnetic soft elastomer films cut into shapes of a body with a tail and wings and a six-legged body (Fig. 1d-h) are presented. Bodies and extremities are discretely magnetized in varying 3D directions (Fig. 1d, e), and magnetization directions are validated by measuring out-of-plane components of their magnetic flux density as shown in insets of Fig. 1d, e.

Upon application of a magnetic field of 60 mT perpendicular to the plane, magnetic torques on components with different magnetization directions try to align them in the direction of the external field, resulting in 3D deformations of the structures (Fig. 1f, g). Furthermore, multicomponent 3D structures can be formed by stacking individual components, as shown in Fig. 1h. The bodies with legs and wings are stacked to form a multicomponent 3D "dragonfly" structure upon magnetic actuation (Fig. 1i).

In Fig. 8, a set of structures with varying 3D magnetization profiles that can transform into complex 3D structures under magnetic fields are presented. A computational model, taking magnetization directions, external magnetic fields, and mechanical deformations into account, was developed to predict the complex 3D shape transformations with the designed magnetization profiles (Fig. 8).

While a ring structure with 4-segmented alternating magnetization profile generates a vertically rising profile upon magnetic actuation (Fig. 8a), a ring of same size with 8-segmented alternating magnetization profile results in undulating edges (Fig. 8b). A 3D half-sphere structure is formed by radially symmetric magnetization of a planar structure composed of hexagonal and pentagonal units connected through hinges (Fig. 8c). Moreover, stacking two of these structures with complementing magnetization profiles enables formation of a closed 3D sphere, which can be also rolled on a planar surface by applying rotating magnetic fields (Fig. 9). Furthermore, other than manual assembly, inherently 3D complex structures can be fabricated out of our magnetic soft elastomers using molding or 3D printing techniques. Formation of closed structures can be also realized by complementary 3D magnetization, thus deformation, of connected segments, as shown in Fig. 1c. Planar segments connected via hinges are magnetized both in-plane and out-of-plane to form a closed cube under a magnetic field of 60 mT (Fig. 8d).

In-situ, i.e. in the lab, magnetic reprogramming of the soft systems is crucial for their optimization, multifunctional operation, and adaptation to dynamic environments. Heat-assisted magnetization strategy allows facile magnetic reprogramming of soft structures on demand.

In Fig. 2a, a "stickman" structure with a 3D magnetization profile encoded in the body, shoulders, arms, and head, which undergoes a 3D complex shape transformation upon magnetic actuation is presented. When the magnetization profile of the stickman structure is reprogrammed, bending of its head and arms can be reconfigured as shown in Fig. 2b,c. Local reprogramming of internal material behavior can also enable the design and the optimization of advanced active metamaterials.

In Fig. 2d, an auxetic mechanical metamaterial structure composed of 8 units is presented. Magnetic programming of the units as shown in Fig. 2e results in expansion and compression of the whole structure both in length and width at the same time, thus displaying a negative Poisson's ratio, depending on the magnetic actuation direction (Fig. 2f,g). Using the heat-assisted magnetic reprogramming approach, magnetization profile of individual units, thus their mechanical behavior, can be reprogrammed (Fig. 10a-g). Reprogramming of multiple units in the middle section of the structure results in expansion in width with minimal change in length, independent of the magnetic actuation direction (Fig. 2h-j).

To further highlight the importance of facile reprogramming, a 4-legged flexible robot with a specific magnetization direction assigned for each leg (Fig. 2k-p, and Fig. 10h-j) is demonstrated. Asymmetric magnetization of legs at two lateral sides generates larger deformation of the right legs and generates a circular trajectory upon magnetic actuation (Fig. 2k-m). Reprogramming magnetization profiles of the soft robot with bilateral and fore-and-aft symmetries result in laterally symmetric deformations of the legs and generate straight trajectories upon magnetic actuation (Fig. 2n-p). These results show that remote and non-invasive reprogramming can be utilized for experimental optimization of the material behavior, such as in mechanical and acoustic metamaterials, and tuning the locomotion performance and characteristics of soft robots.

Heat-assisted magnetization can also be extended for programming complex 3D structures. In Fig. 3a, a 3D printed non-magnetic tree body is presented with magnetic leaves assembled at the tips of branches. Magnetization direction of a leave is programmed by laser-based heating while applying a magnetic field in a desired direction, which results in deformation of only the programmed leave upon magnetic actuation (Fig. 3b).

Sequential programming of all magnetic leaves in the same direction results in actuation of all leaves synchronously in the same direction (Fig. 3c). Furthermore, magnetization direction of individual leaves can be reprogrammed on-demand to generate different configurations (Fig. 3d, and Fig. 11), enabling control over shape-deformation instructions distributed over complex 3D structures. Magnetic reprogramming of 3D structures can enable development of reconfigurable soft machines for adaptive interaction with objects of arbitrary morphologies.

As an example, an adaptive soft gripper composed of 4 fingers made out of magnetic soft elastomers (Fig. 3e) was assembled. Out-of-plane magnetization of fingers results in maximum deflection at the fingertips and enables squeezing of a spherical object (3 mm diameter) below the circumference (Fig. 3f a). On the other hand, a vertically placed cylindrical object (4 mm diameter and 4 mm height) requires a larger contact area with the gripping fingers, which is achieved by concave deflection of the fingers via magnetization profile shown in Fig. 3g. Grasping a more complex example of a car-like object (10.6 mm length, 3 mm width, and 1.75 mm height), with an inner cavity and a gap underneath due to the wheels, is achieved by outward deformation of fingers within the inner cavity and inward deformation of the fingers at the sides (Fig. 3h). Compared with existing approaches, our laser-based heat-assisted magnetization strategy enables on-demand and local reprogramming of 3D magnetic structures with arbitrary magnetization profiles.

Microscale robots and machines hold significant potential for manipulation of the microscopic world with applications ranging from bioengineering to minimally invasive medicine. Magnetically programmed shape deformations can enable a new class of microsystems with advanced locomotion and manipulation capabilities. The heat-assisted magnetization approach presented herein can be scaled down to magnetically program microstructures with a spatial resolution of 38 $\mu$m (Fig. 4).

One route for down-scaling is focusing the NIR laser beam size below 200 $\mu$m by using a microscope objective (Fig. 4a). Using focused laser-heating, a soft structure with 6 petals (150 $\mu$m width, 500 $\mu$m length, and 30 $\mu$m thick) is magnetized in complimentary directions to generate synchronized deformation of petals in reverse directions (Fig. 4b,c). Microscale magnetic programming can be also achieved by placing a photomask on top of the magnetic soft elastomers (Fig. 4d-f). Photomask allows laser to pass through the micropatterned areas of different sizes, with the smallest dimension of 65 $\mu$m (Fig. 15a), thus, decreasing the area of heated region to the patterned area. Using photomask-enabled micropatterned laser-heating, "MPI" letters are magnetically programmed in different sizes and magnetization directions in magnetic soft elastomers, as shown by measured magnetic flux density profile (Fig. 4e-f).

Other than laser-based sequential heating and magnetization, magnetic programming can be also realized by generating the desired magnetic pattern (master) in close proximity to the magnetic soft elastomers (slave) and heating the system globally (Fig. 4g), enabling one-shot magnetization of the whole sample. For creating magnetic masters, polyurethane neodymium-iron-boron (NdFeB) composite magnets, with a higher Curie temperature than $CrO_2$, of different size, shapes, and polarities in different configurations are manually arranged (Fig. 4h, i). Magnetic one-shot pattern transfer is achieved by putting the magnetic soft elastomer slaves in direct contact with the magnetic masters and globally heating to 150 °C (Fig. 4h,i). A magnetic master with a complex "Minerva" symbol (Fig. 4j and Fig. 15b) was also fabricated. By contact magnetic transfer, magnetic profile of the magnetic master is copied into a magnetic soft elastomer slaves, with the smallest dimension of 38 $\mu$m (Fig. 4j). While high-resolution 2D magnetic programming has been previously shown in rigid panels connected via flexible hinges, heat-assisted magnetization strategy described here enables 3D magnetic programming at a comparable resolution (Fig. 12).

Heat-assisted magnetic programming strategy introduced here is inherently decoupled from the fabrication method of the magnetic soft elastomers and enables a non-invasive, i.e. non-surgical and non-destructive, means for reprogramming shape-deformations encoded into the material at high spatial resolutions. Facile and non-invasive magnetic reprogramming can enable rapid and data-based optimization of performance and behavior of soft systems, such as mechanical and optical soft metamaterials and kirigami-enabled structures. Resolution and speed of heat-assisted magnetic programming can be further scaled down using well-established magneto-optical recording techniques used in the data storage industry. Moreover, heat-assisted magnetic contact transfer shown in Fig. 4g-j can be adapted for high-throughput magnetic encoding utilizing a magnetic master and a three-axis stage, which enables programming 10 samples per minute. High-throughput magnetic contact transfer could be further combined with multiple masters of desired magnetic profiles and mechanical punchers to cut the magnetic soft elastomer samples in desired shapes, paving the way for future continuous roll-to-roll mass production of magnetic soft machines (Fig. 12).

Other magnetic particles, with engineered Curie temperatures low enough to sustain operation temperature of polymers, as well as other polymers or gels with softer material properties, can be employed for enhanced material performance. In the present description the focus is on a laser 24 for heating the soft magnetic elastomers, remote and selective heating can be also achieved by remote power transfer to thin receiver coils attached on the elastomers. Application of AC magnetic fields can be also used for global heating along with spatially patterned DC magnetic fields for programming the magnetic soft elastomers. Remote magnetic programming and reprogramming can enable adaptive operation of soft untethered systems in closed and confined dynamic environments. Magnetically responsive multi-scale soft systems with reprogrammable complex shape-transformation capabilities will inspire diverse applications in medical robots, wearable health monitoring pieces of equipment, and bio-inspired microrobots.

In order to prepare the composite, the following can be done:

Preparation of the composite 12 formed by magnetic elastomers:

$CrO_2$ powder (Sigma-Aldrich, St. Louis, MO) was heated for 3 h at 300 °C in an oven. 22 g of baked $CrO_2$ particles were dispersed in 250 mL of sodium bisulfite ($NaHSO_3$) solution (from https://www.sigmaaldrich.com/catalog/product/sigald/243973?lang=de&region=DE) in deionized (DI) water (50 g/L, Sigma-Aldrich, St. Louis, MO) and kept at 65 °C for 16 h while agitated occasionally. Then, the particles were washed 5 times with 1 L DI water and filtered by using a test sieve with a mesh size of 20 $\mu$m. The remaining $CrO_2$ particles were left in a fume hood for two days to remove any remaining water. The resulting film was scraped and crushed using a pestle and mortar to obtain final dried and stabilized $CrO_2$ particles.

$CrO_2$/PDMS magnetic soft elastomer composites were prepared by adding the dried and stabilized $CrO_2$ particles into the siloxane base (Dow Corning, Midland, MI) at 1:2 ($CrO_2$:Siloxane base) mass ratio and shear mixing with a Pasteur pipette for 5 min. Next, the crosslinking agent was added into the pre-polymer mixture at a crosslinking agent to mixture mass ratio of 1:10 and further shear mixed for 5 min. Then, the mixture was cast into molds composed of two tapes of desired thicknesses (25 $\mu$m to 200 $\mu$m) adhered on a flat glass substrate and cured for 4 h at 90 °C. A UV laser system (LPKF ProtoLaser U3, Garbsen, Germany) was used to cut the desired geometries out of the magnetic elastomer films. Thickness of the magnetic elastomer films was measured with an optical profilometer (VK-X250, Keyence, Osaka, Japan). Elastic modulus (E) and strain of the magnetic elastomers were experimentally characterized by uniaxial tensile testing of non-heated and heated dog-bone-shaped samples at a strain rate of 0.005 s$^{-1}$ (Instron 5942, Instron, Norwood, MA).

Once the magnetic soft devices 10 have been formed, one can start with the heat-assisted magnetic (re)programming.

Local heating of $CrO_2$ elastomer films, i.e. of the regions 22 of the device 10, was achieved by using a power-adjustable fiber-coupled NIR laser with a collimator (808 nm, 133-457 mW, Edmund Optics, Barrington, NJ). The temperature and the heated spot size on the magnetic elastomer films were measured using an infrared thermal camera (ETS320, Wilsonville, OR) at 7 cm distance. Heating and cooling times of the magnetic soft elastomers were measured by heating the samples for 100 s. Samples were placed on an automated stage (Axidraw v3, Evil Mad Scientist, Sunnyvale, CA) and NdFeB magnet (20 mm diameter and 20 mm thickness, Supermagnete, Gottmadingen, Germany) that can be rotated 360° were placed underneath the magnetic soft elastomer during heating and cooling to align the magnetization direction of the $CrO_2$ particles (Fig. 14a, b).

Applied magnetic field magnitude and direction were continuously monitored by using a 3D magnetic hall sensor (TLE493D-W2B6, Infineon Technologies, Munich, Germany) and adjusted according to the desired magnetization direction.

Magnetization of the magnetic soft elastomers was measured with a vibrating sample magnetometer (VSM; MicroSense, Lowell, MA). Circular samples with 1 mm diameter were placed on a sample holder and hysteresis loop of $CrO_2$ obtained at external fields ranging from 1.5 T to -1.5 T (Fig. 5a). Magnetization of the magnetic soft elastomers as devices 10 were calculated as 9.8 kA/m, by dividing the remanent magnetization to the sample volume. Magnetization efficiency was determined as the ratio of magnetization of the heat-magnetized samples and samples magnetized under 1.8 T field in VSM. Magnetization profile of the magnetically programmed samples were characterized by measuring the magnetic flux densities at sample surfaces via a magneto-optical sensor (MagViewS, Matesy, Jena, Germany).

In order to design the devices 10 and then compare the designs to the model developed computational modeling of shape deformations was performed. For this purpose, a finite element analysis is employed for predictive modeling of the shape changes under magnetic actuation (Fig. 13).

COMSOL structural mechanics module (COMSOL, Burlington, MA) is linked to a custom MATLAB script (MathWorks, Natick, MA) via "LiveLink". Sample geometries are divided into smaller sub-sections with pre-defined magnetization profiles and MATLAB script is used for calculation of magnetic forces and torques, while mechanical deformations are solved in COMSOL.

After every iteration, magnetic forces and torques were recalculated according to the updated magnetization direction vector for each subsection until a quasi-static equilibrium state in 3D is reached. For all simulations, experimentally measured E of 200 kPa and magnetization of 9.8 kA/m were employed. Density of the magnetic soft elastomer was calculated as 3.89 g/cm$^3$ and Poisson's ratio is assumed 0.49.

In order to magnetically actuated the devices 10 formed a cylindrical NdFeB magnet 44 (60 mm diameter and 10 mm thick, Supermagnete, Gottmadingen, Germany) was used. The magnet 44 was guided towards the devices 10 arranged on the platform 46 in the vertical or horizontal direction for magnetic actuation (Fig. 14c). For magnetic actuation under uniform fields, a Halbach array 48 composed of 16 permanent magnets 50 (10 mm x 10 mm x 10 mm) was used (Fig. 14d). For dynamic actuation, the Hallbach Array 48 was rotated for rotational actuation of the samples.

Once a device 10 had been programmed, Magnetic (re)programming at the micron scale can be carried out. For magnetic (re)programming at the micron scale, three different approaches were employed: focused laser heating, photomask-enabled micropatterned laser heating, and contact magnetic transfer via global heating. Focused laser heating was achieved by placing a microscope objective (20x, NA 0.5, Carl Zeiss, Oberkochen, Germany) in the laser 24 beam path and decreasing the beam size below 200 microns.

For photomask-enabled micropatterned laser heating, a photomask containing microscale patterns (Fig. 15a) was placed on top of the samples with a 20 $\mu$m gap. When exposed to the laser beam, which can only pass through the micropatterned areas, the samples were heated locally in the shape of patterns available on the photomask.

For contact transfer of magnetic profiles, polyurethane NdFeB magnetic composites of different shapes were utilized. First, an SU-8 positive template of desired geometries on a silicon wafer was fabricated by photolithography and wet chemical development. For positive template fabrication, SU-8 100 (Microchem Inc., Newton, MA) was disposed on a silicon wafer, spin-coated at 2500 rpm for 45 s, pre-baked on a hot plate at 95 °C for 30 min, and cooled down to room temperature. Next, the photoresist coated wafer is loaded into a mask aligner (MJB4 Mask Aligner, SUSS MicroTec, Garching, Germany) with a photomask containing desired patterns to be fabricated and exposed to a UV light (365 nm, 13 mW/cm$^2$) for 15 s. Then, photoresist-coated wafers was baked for 10 min at 95 °C, cooled down to room temperature, and immersed in a chemical developer (mr-600, micro resist technology, Berlin, Germany) with slight agitation for approximately 10 minutes and later rinsed in IPA for about 2 minutes. Last, the microfabricated template was baked on a hotplate for 30 min. at 100 °C. Then, silicone rubber (Mold Max 20, Smooth-On, Macungie, PA) was poured over the positive template, cured at room temperature for 4 h, and peeled off, resulting in a negative template. Afterwards, polyurethane pre-polymer (Smooth-Cast 310/1, Smooth-On, Macungie, PA) mixed with NdFeB powder (MQFP-15-7, Magnequench, Toronto, Canada) at 1:1 mass ratio was molded into the negative template and cured for 4 h at room temperature and peeled off.

Prepared polyurethane NdFeB magnets were pre-magnetized and magnetic fields generated by polyurethane NdFeB magnets were smaller than the coercivity of the magnetic soft elastomers. While modular polyurethane magnets were manually arranged in desired configurations, the ones with complex shapes were used as monolithic units. Finally, for contact magnetic transfer, the magnetic soft elastomers were placed on top of polyurethane NdFeB magnets and placed into an oven for 5 min. at 150 °C and cooled down to room temperature while in contact.

In this way a method of encoding a programmable and/or reprogrammable magnetic soft device 10 is made available, the method comprising the steps of:

- heating the composite to a temperature above the Curie temperature of the magnetic elements distributed therein;
- cooling the composite; and
- re-orienting magnetic domains of the magnetic elements by applying an external magnetic field during cooling.

The steps of heating and cooling the composite may be carried out sequentially by sequentially focusing the tunable laser 24 onto regions of said composite and cooling said regions optionally before moving on to further regions of said composite, alternatively they may be carried out only once using e.g. a master as described in the foregoing.

The step of applying the magnetic field may be carried out with a magnetic field having a magnitude selected in the range of 1 mT to 10 T, in particular for each cooling cycle to orient each region of the composite 12 with its own magnetic magnetization profile.

Design of the magnetic soft structures 10 takes both the geometry and the magnetization profile into consideration for controlled shape changing. Intuitive designs can be used for simple shape changes under external magnetic fields, but more demanding and complex deformations require a predictive model. For this reason, a predictive model utilizing COMSOL and a custom MATLAB script to solve for the quasi static state of the magnetic soft structures was developed.

The predictive model is based on the following assumptions: Magnetic soft structures 10 are subjected to magnetic forces (f), magnetic torques ($\tau$), and gravitational forces (mg), which creates stresses on the soft body 10 which deforms to minimize the total magnetic and elastic potential energy. Moreover, direction of magnetic forces and torques changes along with the magnetization direction during deformation, creating a distributed heterogeneous response to the external magnetic fields over the structure. To capture this heterogeneous response, each sample geometry is divided into smaller subsections labeled by '$i$' with the pre-defined dimensions of $d_x$, $d_y$, $d_z$ and magnetic moment of $\boldsymbol{m}^i$ (Fig. 13a, b). Magnetic forces and torques for each subsection are calculated in a custom MATLAB script according to the applied magnetic field (B) and the magnetic moment of each sub-section. Calculated magnetic forces ($\boldsymbol{f}^i = \nabla(\boldsymbol{m}^i \cdot \boldsymbol{B})$) then applied as directional forces to the sub-sections. On the other hand, magnetic torques ($\tau^i = \boldsymbol{m}^i \times \boldsymbol{B}$) are distributed as forces on the facets of the subsections. To achieve this, magnetic torque ($\tau^i$) is separated into its orthogonal components ( $\tau_x^i, \tau_y^i, \tau_z^i$ ) for a chosen Cartesian reference frame (Fig. 13c). Then, these torque components are converted to the facet forces $f_{\tau_x}^i, f_{\tau_y}^i, f_{\tau_z}^i$ (Fig. 13d). The directions of the forces on the facets are determined by following the right-hand rule, while their magnitudes are calculated as follows,

$$\left|f_{\tau_x}^i\right| = \frac{\tau_x^i}{d_z\,/2}\ ,\ \left|f_{\tau_y}^i\right| = \frac{\tau_y^i}{d_x\,/2}\ ,\ \left|f_{\tau_z}^i\right| = \frac{\tau_z^i}{d_y\,/2}\ , \qquad\qquad (Eq.\,1)$$

where $f_{\tau_x}^i, f_{\tau_y}^i, f_{\tau_z}^i$ are the torque induced forces due to the torques $\tau_x^i, \tau_y^i, \tau_z^i$, respectively. These forces are then transferred to the COMSOL via LiveLink, and structural mechanics module is used to solve for the elastic deformation of the magnetic soft structures. After every iteration, magnetic forces and torques are recalculated according to the updated magnetization direction vector in each subsection at deformed state until an equilibrium was reached.

Validation of the model is performed by using a beam structure with dimension of 10 mm length x 1 mm width x 0.17 mm thickness. Beam is magnetized along its long axis and fixed at 1.25 mm from one end. Then, magnetic fields in the range of 0 to 56 mT applied vertical to the magnetization direction of the beam. Both experimental and simulation results are obtained for the same conditions (Fig. 13e). Deflection angle $\theta$ is calculated for both experimental and simulation results and compared (Fig. 13f). The developed computational approach captures the deformation characteristics and is in good agreement with the experimental results.

List of reference numerals:

| | |
|---|---|
| 10 | device |
| 12 | composite |
| 14 | body |
| 16 | legs |
| 18 | wings |
| 19 | tail |
| 20 | sections |
| 22 | regions |
| 24 | laser |
| 26 | Jig |
| 28, 28' | top half of 26, lower half of 26 |
| 30 | permanent magnet |
| 32 | boxes |
| 34 | finger |
| 36 | magnetization setup |
| 38 | motorized stage |
| 40 | hall-effect sensor |
| 42 | collimator |
| 44 | magnet |
| 46 | actuation platform |
| 48 | Halbach Array |
| 50 | magnet |
| 52 | photomask |

**Claims**

1. A method of fabricating a programmable and/or reprogrammable magnetic soft device (10) having a Young's modulus of less than 500 MPa in one or more parts of the device (10), the method comprising the steps of:

> - forming a composite (12) of base material and magnetic elements distributed within said base material;
> - shaping the composite (12) to have a desired final shape;
> - heating the composite (12) while applying or not applying a magnetic field at the composite (12); and
> - cooling the composite (12) while applying a magnetic field at the composite (12), with the step of heating (12) comprising heating the composite (12) to a temperature selected in the range of 25% below the Curie temperature to a temperature 25 % above the Curie temperature measured in °C of said magnetic elements, wherein the melting temperature of the base material is higher than the maximum temperature applied to the magnetic composite (12) during the heating step.

**2.** A method of fabricating a programmable and/or reprogrammable magnetic soft device (10) in accordance with claim 1,
wherein the step of heating is carried out before, and/or after and/or during the step of shaping the composite (12).

**3.** A method of fabricating a programmable and/or reprogrammable magnetic soft device (10) in accordance with claim 1 or claim 2,
wherein the step of shaping and the step of heating are carried out simultaneously.

**4.** A method of fabricating a programmable and/or reprogrammable magnetic soft device (10) in accordance with any one of the preceding claims,
wherein the applied magnetic field during the heating and/or cooling step is below the coercive magnetic field of the magnetic element at its room temperature state.

**5.** A method of fabricating a programmable and/or reprogrammable magnetic soft device (10) in accordance with any one of the preceding claims,
wherein the step of shaping the composite (12) comprises at least one of the following steps; molding the composite (12) in one mold of pre-defined shape and size, molding one or more parts of the composite (12) in one or more molds of same shape and size, molding the composite (12) in one or more molds of differing shapes and sizes, photolithographing the composite (12), photolithographing one or more parts of the composite (12), stereo lithographing the composite (12), stereo lithographing one or more parts of the composite (12), 3D printing the composite (12), 3D printing one or more parts of the composite (12), combining parts of the composite (12), cutting sections of material from the composite (12), cutting sections of material from parts of the composite (12) and combinations of the foregoing.

**6.** A method of fabricating a programmable and/or reprogrammable magnetic soft device (10) in accordance with any one of the preceding claims,
wherein the steps of heating and cooling the composite (12) are carried out a plurality of times sequentially for different regions of the composite (12).

**7.** A method of fabricating a programmable and/or reprogrammable magnetic soft device (10) in accordance with claim 6,
wherein the step of magnetization is carried out for each step of cooling for each region of the composite (12) so that each region is provided with its own magnetization direction.

**8.** A method of fabricating a programmable and/or reprogrammable magnetic soft device (10) in accordance with any one of the preceding claims,

wherein the step of heating the composite (12) is carried out with a light source, in particular a tunable laser, or wherein the step of heating the composite (12) is carried out with one of a convection oven, a hot-plate and a heat-gun; and/or
wherein the steps of heating and cooling the composite (12) are carried out a single time globally for different regions of the composite (12), in particular wherein the step of magnetization is carried out for a single time during cooling for each region of the composite (12) by using a magnetic master configured to generate desired magnetization profile so that each region is provided with its own magnetization direction; and/or
wherein the step of applying the magnetic field is carried out with a magnetic field having a magnitude selected in the range of 1 mT to 10 T.

**9.** A method of encoding a programmable and/or reprogrammable magnetic soft device (10) manufactured in accordance with any one of claims 1 to 8, the method comprising the steps of:

- heating the composite (12) to a temperature selected in the range of 25% below the Curie temperature to a temperature 25 % above the Curie temperature measured in °C of the magnetic elements distributed therein; wherein the melting temperature of the base material is higher than the maximum temperature applied to the magnetic composite (12) during the heating step;
- cooling the composite (12); and
- re-orienting magnetic domains of the magnetic elements by applying an external magnetic field during cooling, or during both heating and cooling.

**10.** A method of encoding a programmable and/or reprogrammable magnetic soft device (10) in accordance with claim 9,

wherein the steps of heating and cooling the composite (12) are carried out sequentially by sequentially focusing a tunable laser onto regions of said composite (12) and cooling said regions optionally before moving on to further regions of said composite; or
wherein the steps of heating and cooling the composite (12) are carried out globally by using a convection oven of said composite (12) and cooling the composite (12) with a magnetic master placed adjacent to the said composite (12).

**11.** A method of encoding a programmable and/or reprogrammable magnetic soft device (10) in accordance with claim 9 or 10, in particular in accordance with claim 9,
wherein the step of applying the magnetic field is carried out with a magnetic field having a magnitude selected in the range of 1 mT to 10 T, in particular for each cooling cycle to orient each region of the composite (12) with its own magnetic magnetization profile.

**Patentansprüche**

**1.** Verfahren zur Herstellung einer programmierbaren und/oder umprogrammierbaren magnetischen weichen Vorrichtung (10) mit einem Elastizitätsmodul von weniger als 500 MPa in einem oder mehreren Teilen der Vorrichtung (10), wobei das Verfahren die Schritte umfasst:

- Bilden eines Verbundwerkstoffs (12) aus Basismaterial und magnetischen Elementen, die innerhalb des Basismaterials verteilt sind;
- Formen des Verbundwerkstoffs (12), so dass er eine gewünschte Endform aufweist;
- Erwärmen des Verbundwerkstoffs (12), während ein Magnetfeld an den Verbundwerkstoff (12) angelegt wird oder nicht; und
- Abkühlen des Verbundwerkstoffs (12), während ein Magnetfeld an den Verbundwerkstoff (12) angelegt wird, wobei der Schritt des Erwärmens (12) das Erwärmen des Verbundwerkstoffs (12) auf eine Temperatur ausgewählt im Bereich von 25 % unter der Curie-Temperatur bis zu einer Temperatur 25 % über der Curie-Temperatur, gemessen in °C der magnetischen Elemente, umfasst, wobei die Schmelztemperatur des Basismaterials höher als die maximale Temperatur sein kann, die während des Erwärmungsschritts an den magnetischen Verbundwerkstoff (12) angelegt wird.

**2.** Verfahren zur Herstellung einer programmierbaren und/oder umprogrammierbaren magnetischen weichen Vorrichtung (10) nach Anspruch 1, wobei der Schritt des Erwärmens vor und/oder nach und/oder während des Schritts des Formens des Verbundwerkstoffs (12) durchgeführt wird.

**3.** Verfahren zur Herstellung einer programmierbaren und/oder umprogrammierbaren magnetischen weichen Vorrichtung (10) nach Anspruch 1 oder Anspruch 2,
wobei der Schritt des Formens und der Schritt des Erwärmens gleichzeitig durchgeführt werden.

**4.** Verfahren zur Herstellung einer programmierbaren und/oder umprogrammierbaren magnetischen weichen Vorrichtung (10) nach einem der vorhergehenden Ansprüche,
wobei das angelegte Magnetfeld während des Erwärmungs- und/oder Abkühlungsschritts unter dem Koerzitivmagnetfeld des magnetischen Elements in seinem Raumtemperaturzustand liegt.

**5.** Verfahren zur Herstellung einer programmierbaren und/oder umprogrammierbaren magnetischen weichen Vorrichtung (10) nach einem der vorhergehenden Ansprüche,
wobei der Schritt des Formens des Verbundwerkstoffs (12) mindestens einen der Schritte umfasst: Formen des Verbundwerkstoffs (12) in einer Form mit vordefinierter Form und Größe, Formen eines oder mehrerer Teile des Verbundwerkstoffs (12) in einer oder mehreren Formen mit gleicher Form und Größe, Formen des Verbundwerkstoffs (12) in einer oder mehreren Formen mit unterschiedlichen Formen und Größen, Fotolithographie des Verbundwerkstoffs (12), Fotolithographie eines oder mehrerer Teile des Verbundwerkstoffs (12), Stereolithographie des Verbundwerkstoffs (12), Stereolithographie eines oder mehrerer Teile des Verbundwerkstoffs (12), 3D-Drucken des Verbundwerkstoffs (12), 3D-Drucken eines oder mehrerer Teile des Verbundwerkstoffs (12), Kombinieren von Teilen des Verbundwerkstoffs (12), Schneiden von Abschnitten von Material aus dem Verbundwerkstoff (12), Schneiden von Abschnitten von Material aus Teilen des Verbundwerkstoffs (12) und Kombinationen der Vorstehenden.

**6.** Verfahren zur Herstellung einer programmierbaren und/oder umprogrammierbaren magnetischen weichen Vorrichtung (10) nach einem der vorhergehenden Ansprüche,
wobei die Schritte des Erwärmens und Abkühlens des Verbundwerkstoffs (12) mehrere Male nacheinander für verschiedene Bereiche des Verbundwerkstoffs (12) durchgeführt werden.

**7.** Verfahren zur Herstellung einer programmierbaren und/oder umprogrammierbaren magnetischen weichen Vorrichtung (10) nach Anspruch 6, wobei der Schritt der Magnetisierung für jeden Schritt des Abkühlens für jeden Bereich des Verbundwerkstoffs (12) durchgeführt wird, so dass jeder Bereich mit seiner eigenen Magnetisierungsrichtung versehen ist.

**8.** Verfahren zur Herstellung einer programmierbaren und/oder umprogrammierbaren magnetischen weichen Vorrichtung (10) nach einem der vorhergehenden Ansprüche,

wobei der Schritt des Erwärmens des Verbundwerkstoffs (12) mit einer Lichtquelle, insbesondere einem abstimmbaren Laser, durchgeführt wird, oder
wobei der Schritt des Erwärmens des Verbundwerkstoffs (12) mit einem von einem Konvektionsofen, einer Heizplatte und einer Heizkanone durchgeführt wird; und/oder
wobei die Schritte des Erwärmens und Abkühlens des Verbundwerkstoffs (12) ein einziges Mal global für verschiedene Bereiche des Verbundwerkstoffs (12) durchgeführt werden, insbesondere
wobei der Schritt der Magnetisierung ein einziges Mal während des Abkühlens für jeden Bereich des Verbundwerkstoffs (12) unter Verwendung eines magnetischen Masters durchgeführt wird, der konfiguriert ist, um ein gewünschtes Magnetisierungsprofil zu erzeugen, so dass jeder Bereich mit seiner eigenen Magnetisierungsrichtung versehen ist; und/oder wobei der Schritt des Anlegens des Magnetfelds mit einem Magnetfeld mit einer Größe durchgeführt wird, die im Bereich von 1 mT bis 10 T ausgewählt ist.

**9.** Verfahren zum Codieren einer programmierbaren und/oder umprogrammierbaren magnetischen weichen Vorrichtung (10), die nach einem der Ansprüche 1 bis 8 hergestellt wird, wobei das Verfahren die Schritte umfasst:

- Erwärmen des Verbundwerkstoffs (12) auf eine Temperatur ausgewählt im Bereich von 25 % unter der Curie-Temperatur bis zu einer Temperatur 25 % über der Curie-Temperatur, gemessen in °C der darin verteilten magnetischen Elemente, wobei die Schmelztemperatur des Basismaterials höher als die maximale Temperatur sein kann, die während des Erwärmungsschritts an den magnetischen Verbundwerkstoff (12) angelegt wird;
- Abkühlen des Verbundwerkstoffs (12); und
- Neuausrichten von magnetischen Domänen der magnetischen Elemente durch Anlegen eines externen Magnetfelds während des Abkühlens oder sowohl während des Erwärmens als auch während des Abkühlens.

**10.** Verfahren zum Codieren einer programmierbaren und/oder umprogrammierbaren magnetischen weichen Vorrichtung (10) nach Anspruch 9, wobei die Schritte des Erwärmens und Abkühlens des Verbundwerkstoffs (12) nacheinander durchgeführt werden, indem ein abstimmbarer Laser nacheinander auf Bereiche des Verbundwerkstoffs (12) fokussiert wird und die Bereiche optional gekühlt werden, bevor zu weiteren Bereichen des Verbundwerkstoffs übergegangen wird; oder
wobei die Schritte des Erwärmens und Abkühlens des Verbundwerkstoffs (12) global durchgeführt werden, indem ein Konvektionsofen des Verbundwerkstoffs (12) verwendet wird und der Verbundwerkstoff (12) mit einem magnetischen Master gekühlt wird, der neben dem Verbundwerkstoff (12) platziert ist.

**11.** Verfahren zum Codieren einer programmierbaren und/oder umprogrammierbaren magnetischen weichen Vorrichtung (10) nach Anspruch 9 oder Anspruch 10, insbesondere nach Anspruch 9,
wobei der Schritt des Anlegens des Magnetfelds mit einem Magnetfeld mit einer Größe, die im Bereich von 1 mT bis 10 T ausgewählt ist, insbesondere für jeden Abkühlungszyklus, durchgeführt wird, um jeden Bereich des Verbundwerkstoffs (12) mit seinem eigenen magnetischen Magnetisierungsprofil auszurichten.

## Revendications

**1.** Procédé de fabrication d'un dispositif magnétique souple programmable et/ou reprogrammable (10) ayant un module d'Young inférieur à 500 MPa dans une ou plusieurs parties du dispositif (10), le procédé comprenant les étapes suivantes :

- formation d'un composite (12) de matériau de base et d'éléments magnétiques répartis dans ledit matériau de base ;
- mise en forme du composite (12) pour obtenir une forme finale souhaitée ;
- chauffage du composite (12) tout en appliquant ou en n'appliquant pas un champ magnétique au composite (12) ; et
- refroidissement du composite (12) tout en appliquant un champ magnétique au composite (12), l'étape de chauffage (12) consistant à chauffer le composite (12) à une température choisie dans la plage comprise entre 25 % en dessous de la température de Curie et 25 % au-dessus de la température de Curie mesurée en °C desdits éléments magnétiques, dans lequel la température de fusion du matériau de base est supérieure à la température maximale appliquée au composite magnétique (12) pendant l'étape de chauffage.

2. Procédé de fabrication d'un dispositif magnétique souple programmable et/ou reprogrammable (10) selon la revendication 1,
dans lequel l'étape de chauffage est effectuée avant et/ou après et/ou pendant l'étape de mise en forme du composite (12).

3. Procédé de fabrication d'un dispositif magnétique souple programmable et/ou reprogrammable (10) selon la revendication 1 ou la revendication 2,
dans lequel l'étape de mise en forme et l'étape de chauffage sont effectuées simultanément.

4. Procédé de fabrication d'un dispositif magnétique souple programmable et/ou reprogrammable (10) selon l'une quelconque des revendications précédentes,
dans lequel le champ magnétique appliqué pendant l'étape de chauffage et/ou de refroidissement est inférieur au champ magnétique coercitif de l'élément magnétique à son état à température ambiante.

5. Procédé de fabrication d'un dispositif magnétique souple programmable et/ou reprogrammable (10) selon l'une quelconque des revendications précédentes,
dans lequel l'étape de mise en forme du composite (12) comprend au moins l'une des étapes suivantes : moulage du composite (12) dans un moule de forme et de taille prédéfinies, moulage d'une ou plusieurs parties du composite (12) dans un ou plusieurs moules de même forme et de même taille, moulage du composite (12) dans un ou plusieurs moules de formes et de tailles différentes, photolithographie du composite (12), photolithographie d'une ou plusieurs parties du composite (12), stéréolithographie du composite (12), stéréolithographie d'une ou plusieurs parties du composite (12), impression 3D du composite (12), impression 3D d'une ou plusieurs parties du composite (12), combinaison de parties du composite (12), découpe de sections de matériau à partir du composite (12), découpe de sections de matériau à partir de parties du composite (12) et combinaisons de ce qui précède.

6. Procédé de fabrication d'un dispositif magnétique souple programmable et/ou reprogrammable (10) selon l'une quelconque des revendications précédentes,
dans lequel les étapes de chauffage et de refroidissement du composite (12) sont effectuées plusieurs fois de manière séquentielle pour différentes régions du composite (12).

7. Procédé de fabrication d'un dispositif magnétique souple programmable et/ou reprogrammable (10) selon la revendication 6,
dans lequel l'étape de magnétisation est effectuée pour chaque étape de refroidissement pour chaque région du composite (12) de sorte que chaque région soit pourvue de sa propre direction de magnétisation.

8. Procédé de fabrication d'un dispositif magnétique souple programmable et/ou reprogrammable (10) selon l'une quelconque des revendications précédentes,

dans lequel l'étape de chauffage du composite (12) est effectuée à l'aide d'une source lumineuse, en particulier un laser accordable, ou
dans lequel l'étape de chauffage du composite (12) est effectuée à l'aide d'un four à convection, d'une plaque chauffante ou d'un pistolet thermique ; et/ou
dans lequel les étapes de chauffage et de refroidissement du composite (12) sont effectuées une seule fois globalement pour différentes régions du composite (12), en particulier dans lequel l'étape de magnétisation est effectuée une seule fois pendant le refroidissement pour chaque région du composite (12) en utilisant un maître magnétique configuré pour générer le profil de magnétisation souhaité de sorte que chaque région soit pourvue de sa propre direction de magnétisation ; et/ou

dans lequel l'étape d'application du champ magnétique est effectuée avec un champ magnétique ayant une intensité choisie dans la plage de 1 mT à 10 T.

9. Procédé de codage d'un dispositif magnétique souple programmable et/ou reprogrammable (10) fabriqué selon l'une quelconque des revendications 1 à 8, le procédé comprenant les étapes suivantes :

- chauffage du composite (12) à une température choisie dans la plage comprise entre 25 % en dessous de la température de Curie et 25 % au-dessus de la température de Curie mesurée en °C des éléments magnétiques qui y sont répartis ;
dans lequel la température de fusion du matériau de base est supérieure à la température maximale appliquée au composite magnétique (12) pendant l'étape de chauffage ;
- refroidissement du composite (12) ; et
- réorientation de domaines magnétiques des éléments magnétiques en appliquant un champ magnétique externe pendant le refroidissement, ou pendant à la fois le chauffage et le refroidissement.

10. Procédé de codage d'un dispositif magnétique souple programmable et/ou reprogrammable (10) selon la revendication 9,

dans lequel les étapes de chauffage et de refroidissement du composite (12) sont effectuées séquentiellement en focalisant séquentiellement un laser accordable sur des régions dudit composite (12) et en refroidissant lesdites régions facultativement avant de passer à d'autres régions dudit composite ; ou
dans lequel les étapes de chauffage et de refroidissement du composite (12) sont effectuées globalement en utilisant un four à convection dudit composite (12) et en refroidissant le composite (12) avec un maître magnétique placé de manière adjacente audit composite (12).

11. Procédé de codage d'un dispositif magnétique souple programmable et/ou reprogrammable (10) selon la revendication 9 ou 10, en particulier selon la revendication 9,
dans lequel l'étape d'application du champ magnétique est effectuée avec un champ magnétique ayant une intensité choisie dans la plage de 1 mT à 10 T, en particulier pour chaque cycle de refroidissement afin d'orienter chaque région du composite (12) avec son propre profil de magnétisation magnétique.

# Fig.1

a

14

Magnetic soft elastomer

Laser Heating (808 nm)  24

12  10

Permanent Magnet

22  22

T=R.T.

22

T>T$_C$

T=R.T.

22

b

$\Delta t(118°C)=1.7s$

$\Delta t(59°C)=4s$

175
125
75
25
253mW

50  100  150
Time (s)

Temperature (°C)

c

100

50

0

Magnetization (%)

Non-magnetized  Head-magnetized  Head-demagnetized

2mT

-2mT

d

19  10

18  18

18  18

14

f

10

B

16

16  14

h

9  10  18

18

2mT

-2mT

g

e  16

16

16

16  14  10

10

10

B

16

14

16

i

18  10

18

B

10  16

10

Fig.2

# Fig.3

# Fig.4

Method I: Focused laser heating

a  Objective

B↑

MSE

b

c  10  34  Petales

150µm  Post

34  10  34

B↑  B↓

34  34  10  34

Method II: Micropatterned laser heating

d  Photomask

10  MPI  MPI

B↑  MSE

22

e  250µm  MPI  MPI

f  250µm  MPI

2mT

-2mT

Method III: Contact magnetic transfer

g  MSE  →  Global Heating (T > T_c)

NdFeB Magnets

h  Magnetic Master  1mm

Magnetic Master  1mm

MSE Salve  1mm

-4mT  4mT

i  Magnetic Master  1mm

Magnetic Master  1mm

Magnetic Master

-4mT  4mT

j  Magnetic Master  1mm

MSE Salve

-2mT  2mT

250µm

# Fig.5

a  Thickness (µm)  200  150  100  50

25µm  50µm  180µm
Mold Thickness

b  E (MPa)  0.20  0.15  0.10  0.05  0.00

Without heating  With heating

c  Strain (%)  175  150  125  100  75  50

Without heating  With heating

Fig.6

Fig.7

Fig.8

Fig.9

## Fig.10

## Fig.11

## Fig.12

Fig.13

Fig.14

a

Collimator
x-y stage

42  34  36  38

Fiber optic cable

40

Permanent magnet

Laser source

b  3D magnetic Hall sensor  34  Laser beam  36  40

S
N

30

c

10

Platform  46

Vertical Motion  Magnet  Rotational Motion

44  Horizontal Motion

d

Magnets

50

48

Fig.14e

28

22  10

26

22

28'

Fig.14f

→ B̄

28

10

28'

→ B̄

26

Fig.15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **KIM et al.** Printing ferromagnetic domains for untethered fast-transforming soft materials. *Nature*, vol. 558
- **HU, W.** ; **LUM, G. Z.** ; **MASTRANGELI, M.** ; **SITTI, M.** Small-scale soft-bodied robot with multimodal locomotion.. *Nature*, 2018, vol. 554, 81-85
- **XU, T.** ; **ZHANG, J.** ; **SALEHIZADEH, M.** ; **ONAIZAH, O.** ; **DILLER, E.** Millimeter-scale flexible robots with programmable three-dimensional magnetization and motions.. *Science Robotics*, 2019, vol. 4, 4494

- **CUI, J. et al.** Nanomagnetic encoding of shape-morphing micromachines.. *Nature*, 2019, vol. 575, 164-168
- **KIM, Y.** ; **YUK, H.** ; **ZHAO, R.** ; **CHESTER, S. A.** ; **ZHAO, X.** Printing ferromagnetic domains for untethered fast-transforming soft materials.. *Nature*, 2018, vol. 558, 274-279